# EUROPÄISCHE PATENTSCHRIFT

(11) **EP 3 000 535 B1**
(45) Veröffentlichungstag und Bekanntmachung des Hinweises auf die Patenterteilung: **17.05.2017**
(21) Anmeldenummer: 15186223.2
(22) Anmeldetag: 22.09.2015
(51) Int. Cl.: B05C 17/005, B65D 81/32, B05C 17/015, A61B 17/88

(54) **PASTEN-APPLIKATIONSSYSTEM ZUM MISCHEN EINER PASTE AUS ZWEI KOMPONENTEN**
PASTE APPLICATION SYSTEM FOR THE MIXING OF A PASTE MADE OF TWO COMPONENTS
SYSTEME D'APPLICATION DE PATE DESTINE AU MELANGE D'UNE PATE A PARTIR DE DEUX COMPOSANTS

(30) Priorität: 24.09.2014 DE 102014113816
(43) Veröffentlichungstag der Anmeldung: 30.03.2016
(73) Patentinhaber: Heraeus Medical GmbH, 61273 Wehrheim (DE)
(72) Erfinder: Dr. Vogt, Sebastian, 99092 Erfurt (DE); Greiner, Clemens, 30826 Garbsen (DE)
(74) Vertreter: Heraeus IP

(56) Entgegenhaltungen:
- EP-A1- 0 213 073
- WO-A2-2007/028253
- US-A1- 2011 160 737
- US-B2- 8 579 908

## Beschreibung

Die Erfindung betrifft ein Pasten-Applikationssystem zum Lagern zweier Ausgangskomponenten, zum Mischen der Ausgangskomponenten zu einer Paste und zum Applizieren der Paste.

Die Erfindung betrifft ferner ein Verfahren zum Mischen und Austreiben einer Paste.

Gegenstand der Erfindung ist somit eine manuell zu betätigende Vorrichtung (das Pasten-Applikationssystem), die zum Austragen von pastenförmigen Massen, insbesondere von pastenförmigem Polymethylmethacrylat-Zementteig (PMMA-Zementteig) bestimmt ist. Das Pasten-Applikationssystem und das Verfahren sind zudem zum Lagern, Vermischen und zum Austragen von pastenförmigen Zweikomponentensystemen vorgesehen.

Polymethylmethacrylat-Knochenzemente (PMMA-Knochenzemente) können nach Vermischung eines Zementpulvers als erster Ausgangskomponente mit einer flüssigen Monomerkomponente als zweiter Ausgangskomponente im noch nicht ausgehärteten, pastenförmigen Zustand als sogenannter Zementteig appliziert werden. Bei Verwendung von Mischsystemen befindet sich der Zementteig im Fall von Pulver-Flüssigkeitszementen in einer Kartusche. Aus dieser Kartusche wird der Zementteig durch Bewegung eines Förderkolbens heraus gedrückt. Die Bewegung des Förderkolbens wird durch mechanische Austragsvorrichtungen bewirkt.

Bei pastenförmigen Zweikomponenten-Knochenzementen werden beide pastenförmigen Komponenten in zwei separaten Kartuschen mit zwei separaten Förderkolben aufbewahrt. Bei der Applikation werden beide Pasten durch Bewegung der Förderkolben aus den Kartuscheninnenräumen in einen statischen Mischer gepresst und nach erfolgter Vermischung durch ein Austragsrohr ausgetragen.

Die Applikation von pastenförmigen Kleb- und Dichtstoffen erfolgt in der prinzipiell gleichen Weise mit Pasten-Applikationssystemen.

Zum Auspressen von zähen viskosen Massen werden gegenwärtig Pasten-Applikationssysteme verwendet, die manuell oder pneumatisch oder auch elektrisch angetrieben werden. Übliche mechanische Pasten-Applikationssysteme nutzen insbesondere Klemmstangen zum Auspressen, die durch einen manuell zu betätigenden Kipphebel angetrieben werden.

Pneumatische Pasten-Applikationssysteme erfordern einen Druckluftanschluss. Dazu sind Druckluftschläuche notwendig, welche die Bewegung des Anwenders und die Anwendung des Pasten-Applikationssystems behindern können. Alternativ dazu ist auch die Verwendung von Druckgaspatronen zur Bereitstellung von Druckgas möglich. Exemplarisch dafür seien die Dokumente US 2,818,999 A, EP 0 213 073 A1 und EP 1 118 313 A1 genannt.

Elektrisch angetriebene Auspressvorrichtungen können sowohl mit Akkumulatoren beziehungsweise mit Batterien als auch mit Hilfe einer stationären Stromversorgung angetrieben werden. Diese Vorrichtungen können mit ihren zum Teil sehr großen Kräften besonders zähe pastenförmige Massen auspressen. Nachteilig ist jedoch bei der Verwendung von Elektromotoren, dass diese kostenintensiv sind und Buntmetalle enthalten. Zudem sind starke Motoren und die Energiespeicher hierfür meist schwer und unhandlich.

Die US 6,582,446 B1, die US 8,579,908 B2, die WO 2007/028253 A2 und die US 2011/0160737 A1 offenbaren Vorrichtungen, die zum Austragen von Knochenzement zur Vertebroplastie und Kyphoplastie, der Augmentation von frakturierten Wirbelkörpern mit Polymethylmethacrylat-Knochenzement, bestimmt sind. Für die Auffüllung von frakturierten Wirbeln werden nur kleine Volumina von wenigen Kubikzentimetern Knochenzement benötigt. Daher sind die beschriebenen Vorrichtungen nur für den Austrag von kleinen Zementvolumina ausgelegt. Bei den bekannten Hydraulikvorrichtungen wird vor der Zementapplikation die pulverförmige Komponente mit der flüssigen Monomerkomponente außerhalb der Hydraulikvorrichtung vermischt und der gebildete Knochenzementteig danach in die Applikationsvorrichtung gefüllt. Die Vertebroplastie und auch die Kyphoplastie erfolgen unter ständiger Röntgenkontrolle. Zur Vermeidung von unnötiger Strahlenbelastung des anwendenden Arztes erfolgt deshalb der Druckaufbau durch den Arzt räumlich getrennt von dem Zementaustrag. Dazu ist ein Handstück mit dem Pumpkolben durch eine flexible Leitung mit dem Applikator verbunden. Der Arzt kann dadurch außerhalb des Röntgenstrahls den Applikator ohne Strahlenbelastung betätigen. Das System muss mit fertig gemischtem Knochenzement bestückt werden. Zudem sind größere Mengen Knochenzements mit dem System nicht anwendbar. Bei sehr zähen Pasten-Mischungen können die bekannten Pasten-Applikationssysteme versagen.

Die Aufgabe der Erfindung besteht darin, die Nachteile des Stands der Technik zu überwinden. Insbesondere soll ein Pasten-Applikationssystem und ein Verfahren zum Mischen und Austreiben einer Paste gefunden werden, mit dem auch eine sehr zähe Masse mit einem einfachen Aufbau gelagert, gemischt und ausgetragen werden kann. Dabei soll das Pasten-Applikationssystem derart kostengünstig sein, um eine einmalige Anwendung (Wegwerfartikel) zu ermöglichen. Wegwerfartikel sind aufgrund der hohen hygienischen Anforderungen im OP-Bereich vorteilhaft.

Eine Aufgabe der Erfindung ist dabei ferner darin zu sehen, ein Pasten-Applikationssystem und ein Verfahren bereit zu stellen, die ohne Verwendung von externen stationären Energiequellen, wie Druckluft oder elektrischen Strom, angetrieben werden kann und die in der Lage ist, zähe pastöse Massen auszupressen. Weiterhin soll die Vorrichtung keine internen Energiequellen, wie Akkumulatoren, Batterien oder Druckgaspatronen, enthalten. Die Austragsvorrichtung soll auch kein Kupfer und auch keine Kupferlegierungen enthalten. Die Austragsvorrichtung soll manuell durch den Anwender ortsunabhängig angetrieben werden können. Die Austragsvorrichtung soll einen maximal vereinfachten Aufbau besitzen. Dadurch soll es möglich sein, eine kostengünstige Austragsvorrichtung bereit zu stellen, die nur zum einmaligen Gebrauch bestimmt ist. Weiterhin soll die Erfindung auch die Aufgabe lösen, ein Verfahren zum Austragen von pastenförmigen Massen mit der zu entwickelnden Austragsvorrichtung zu entwickeln.

Die Aufgabe der Erfindung kann auch darin gesehen werden, ein kompaktes Pasten-Applikationssystem zu entwickeln, bei dem durch manuellen Antrieb eine Vermischung von zähen Zementpasten mit Hilfe eines statischen Mischers möglich ist. Im Pasten-Applikationssystem soll weiterhin vor der Zementapplikation eine separate Lagerung von zwei Zementpasten möglich sein. Das System muss einen manuell angetriebenen Austrag von zumindest 40 g bis 75 g gemischter Zementpaste zulassen. Die Oberfläche des Pasten-Applikationssystems muss weiterhin mit Ethylenoxid zu sterilisieren sein. Wichtig ist, dass während der Applikation durch den erforderlichen hohen Druck keine unerwünschten Zementaustritte möglich sind, die zur Kontamination des medizinischen Anwenders und des Operations-Saals (OP-Saals) führen könnten. Das Pasten-Applikationssystem sollte möglichst mit geringem Kraftaufwand manuell zu betätigen sein. Für die Anwendung ist es weiterhin wichtig, dass beim Stoppen des Zementaustrags, der Zementteig beziehungsweise die Paste nicht in größeren Mengen nachfließt.

Die Aufgaben der Erfindung werden gelöst durch ein Pasten-Applikationssystem zum Lagern zweier Ausgangskomponenten, zum Mischen der Ausgangskomponente zu einer Paste und zum Applizieren der Paste aufweisend
eine Zweikomponentenkartusche mit zwei axial verschiebbaren Förderkolben,
einen ersten Hohlzylinder mit einer offenen Stirnseite und einer zumindest teilweise geschlossenen Stirnseite, in dem sich ein axial beweglicher erster Kolben mit zwei daran befestigten Stößeln befindet und der mit der offenen Stirnseite axial an der Zweikomponentenkartusche anliegend angeordnet ist,
einen zweiten Hohlzylinder, der einen manuell verschiebbaren zweiten Kolben enthält, und
einen Flüssigkeitsbehälter, wobei der zweite Hohlzylinder über ein erstes Leitungsmittel und über ein erstes Rückschlagventil mit dem Flüssigkeitsbehälter flüssigkeitsdurchlässig verbunden oder verbindbar ist und der erste Hohlzylinder über ein zweites Leitungsmittel und über ein zweites Rückschlagventil mit dem zweiten Hohlzylinder flüssigkeitsdurchlässig verbunden oder verbindbar ist,
wobei die Zweikomponentenkartusche und der axial benachbart angeordnete erste Hohlzylinder mit den axial beweglichen Stößeln in einem Druckbehälter angeordnet sind und
wobei in dem Pasten-Applikationssystem ein Hohlraum für die Flüssigkeit durch den Flüssigkeitsbehälter, das erste Leitungsmittel, das zweite Leitungsmittel, das erste Rückschlagventil, das zweite Rückschlagventil, den durch den ersten Kolben begrenzten Innenraum des ersten Hohlzylinders und den durch den zweiten Kolben begrenzten Innenraum des zweiten Hohlzylinders gebildet ist und eine Flüssigkeit in dem Hohlraum für die Flüssigkeit enthalten ist oder in den Hohlraum für die Flüssigkeit einfüllbar ist.

Die Flüssigkeit sollte inkompressibel und zumindest weitgehend frei von Gaseinschlüssen sein, damit ein Druck gut hydraulisch mit der Flüssigkeit übertragen werden kann und damit beim Stoppen des Vortriebs keine größeren Mengen der gemischten Paste, insbesondere des Knochenzements, nachfließen können, weil komprimierte Gaseinschlüsse sich nach Wegfall des manuell aufgebauten Drucks ausdehnen. Als Flüssigkeiten können grundsätzlich alle Flüssigkeiten verwendet werden, die bei 23°C und Normaldruck fließfähig sind. Dabei können auch fließfähige Gele verwendet werden, die volumenstabil aber nicht formstabil sind. Als Flüssigkeiten sind Wasser, Wasser enthaltende Polymergele, niedermolekulares Polyethylenglykol, niedermolekulares Polypropylenglykol, Paraffin und Silikonöle bevorzugt. Besonders bevorzugt sind mit Polymeren verdickte wässrige Lösungen, die einen Schmelzpunkt kleiner -10 °C haben. Zur Senkung des Schmelzpunkts der Flüssigkeit können als Additive Glycerin, 1,2-Ethylenglkol, 1,2-Propylenglykol, 1,3-Propylenglykol, 1,4-Butandiol, Diethylenglykol, Triethylenglykol und Tetraethylenglykol zugesetzt werden. Daneben kommen als Flüssigkeiten auch sonstige nicht toxische und nichtkorrosive Flüssigkeiten oder leicht fließende Gele oder Gelpartikelsuspensionen in Betracht, die einen Schmelzpunkt von kleiner -10°C, besonders bevorzugt von -20°C und ganz besonders bevorzugt von -30°C aufweisen.

Unter einem Rückschlagventil werden alle in eine erste Flussrichtung öffnenden und in eine entgegengesetzte zweite Flussrichtung sperrenden Ventilformen verstanden, wie beispielsweise auch Rückschlagklappen oder federbelastete Rückschlagarmaturen. Besonders bevorzugt sind dabei Kugelrückschlagventile mit Kegelsitz oder Kugelsitz, bei denen eine Kugel mit einer gespannten Druckfeder auf den Sitz gepresst wird, wobei die Druckfeder mit einem Flüssigkeitsstrom beziehungsweise einem Flüssigkeitsdruck weiter komprimiert werden kann, sich dadurch die Kugel vom Sitz löst und dadurch das Ventil öffnet, solange eine Flüssigkeit mit ausreichendem Druck durch das Kugelrückschlagventil strömt. Bei einer entgegengesetzten Strömung sperrt das Kugelrückschlagventil, da der Flüssigkeitsdruck und die Druckfeder die Kugel in den Sitz pressen und dadurch die im Sitz befindliche Öffnung verschließen.

Als Leitungsmittel können alle Flüssigkeitsleitungen verwendet werden, die den Druck der zu leitenden Flüssigkeit unbeschadet überstehen können. Dabei werden also insbesondere druckstabile Hydraulikleitungen bevorzugt.

Eine Zweikomponentenkartusche umfasst zumindest zwei Kartuscheninnenräume für zwei Ausgangskomponenten der Paste. Die Zweikomponentenkartusche kann auch noch weitere Kartuscheninnenräume für die beiden Komponenten oder für zusätzliche dritte Komponenten aufweisen. Eine Dreikomponentenkartusche würde im Sinne der vorliegenden Erfindung also eine Zweikomponentenkartusche umfassen. In gleicher Weise können auch mehr als nur zwei Förderkolben in einer erfindungsgemäßen Zweikomponentenkartusche enthalten sein und an dem ersten Kolben können demzufolge auch mehr als nur zwei Stößel befestigt sein, um die Förderkolben anzutreiben, ohne dass dies aus der vorliegenden Erfindung führt. Der Fachmann erkennt das zugrundeliegende Prinzip und kann es ohne weiteres auf eine größere Anzahl von Kartuschen, Förderkolben, Hohlzylindern, Ventilen, Leitungen und Stößeln übertragen, ohne vom zugrundeliegenden Erfindungsgedanken abzuweichen.

Im Lagerzustand des Pasten-Applikationssystems sind der Flüssigkeitsbehälter, das erste Leitungsmittel, das zweite Leitungsmittel, der zweite Hohlzylinder und der noch nicht expandierte zweite Hohlraum des ersten Hohlzylinders noch nicht zwangsläufig mit Flüssigkeit gefüllt. Die Flüssigkeit kann eingefüllt werden, bevor das Pasten-Applikationssystem angewendet wird. Es ist jedoch auch möglich, dass die Flüssigkeit bereits in dem Pasten-Applikationssystem enthalten ist.

Das erfindungsgemäße Pasten-Applikationssystem ist für das Austragen von Polymethylmethacrylat-Knochenzement, Klebstoffen und auch Dichtstoffen bestimmt und geeignet und kann zum Austrag beliebiger pastöser Massen verwendet werden.

Der erste Hohlzylinder und der zweite Hohlzylinder sind vorzugsweise aus Polyamid, Polyetheramid, Polyimid, Polyethersulfon, Polyketon oder glasfaserverstärkten Kompositen dieser Polymere gefertigt. Daneben ist es auch möglich, den ersten Hohlzylinder und den zweiten Hohlzylinder aus Aluminium oder auch Aluminiumlegierungen herzustellen. Je nach Anwendung sollten gegebenenfalls die Innenwände der Zweikomponentenkartusche mit einem speziellen Kunststoff beschichtet sein, der eine chemische Reaktion der Ausgangskomponenten mit dem Material der Kartuschen verhindert. Das erste und das zweite Leitungsmittel können aus gewebeverstärktem Elastomer oder auch aus Aluminium oder Aluminiumlegierungen bestehen. Die übrigen Bestandteile der Austragsvorrichtung bis auf den Druckbehälter bestehen bevorzugt aus normalen technischen Kunststoffen.

Der Flüssigkeitsbehälter weist vorzugsweise ein größeres Volumen als der erste Hohlzylinder auf.

Der Druckbehälter ist vorzugsweise zylindrisch, um die wirkenden Kräfte gut aufnehmen zu können. Bevorzugt weist der Innenraum des Druckbehälters zylindrische Bereiche auf. Die Form des Druckbehälters muss zumindest bereichsweise an die äußere Form der Zweikomponentenkartusche und an die äußere Form des ersten Hohlzylinders angepasst sein, um die auftretenden Kräfte gut aufnehmen zu können.

Bei erfindungsgemäßen Pasten-Applikationssystemen kann auch vorgesehen sein, dass das Pasten-Applikationssystem ein Austragsrohr mit einem integrierten statischen Mischer aufweist, das an dem den Förderkolben gegenüberliegenden Ende der Zweikomponentenkartusche angeordnet ist oder zu befestigen ist.

Bevorzugt kann das Austragsrohr anstelle eines Verschlusses in das vordere Ende der Zweikomponentenkartusche eingeschraubt beziehungsweise befestigt werden, wobei der Verschluss die beiden Ausgangskomponenten voneinander trennt. Dazu bildet der Verschluss bevorzugt ein Teilstück der Wandungen der Kartuscheninnenräume, so dass die Inhalte der Kartuscheninnenräume beim Herausnehmen beziehungsweise Entfernen des Verschlusses miteinander gemischt werden können.

Des Weiteren kann vorgesehen sein, dass der Druckbehälter ein Zug-Modul nach EN ISO 527 größer 1500 MPa aufweist.

Hierdurch kann sichergestellt werden, dass der Druckbehälter die zum Auspressen und Mischen von zähen PMMA-Knochenzementen notwendigen Drucke zerstörungsfrei aufnehmen kann.

Mit einer Weiterentwicklung des Pasten-Applikationssystems wird auch vorgeschlagen, dass der Flüssigkeitsbehälter ein Volumenausgleichselement aufweist, bevorzugt einen axial verschiebbaren dritten Kolben als Volumenausgleichselement aufweist.

Hierdurch wird erreicht, dass sich kein Unterdruck in dem Flüssigkeitsbehälter aufbaut, wenn Flüssigkeit aus dem Flüssigkeitsbehälter in den ersten Hohlzylinder gepumpt wird. Ein Unterdruck würde sich nachteilig auswirken, da mit der Pumpbewegung zusätzlich Arbeit in den Unterdruck geleistet werden müsste, der in dem Flüssigkeitsbehälter erzeugt würde.

Dabei kann vorgesehen sein, dass an der von dem Bereich für die Flüssigkeit abgewandten Seite des Volumenausgleichselements des Flüssigkeitsbehälters, insbesondere an dem dritten Kolben, eine gespannte Druckfeder angeordnet ist, die das Volumenausgleichselement in Richtung des Flüssigkeitsbehälters drückt, insbesondere den dritten Kolben axial im Flüssigkeitsbehälter bewegt, und Flüssigkeit durch das erste Leitungsmittel und durch das erste Rückschlagventil zum zweiten Hohlzylinder presst.

Hierdurch wird erreicht, dass keine zusätzliche Kraft für den Transport der Flüssigkeit aus dem Flüssigkeitsbehälter in den zweiten und den ersten Hohlzylinder aufgewendet werden muss.

Mit einer Weiterbildung der Erfindung wird auch vorgeschlagen, dass eine Öffnung in der Mantelfläche des Druckbehälters vorgesehen ist, die mit einer Öffnung im ersten Hohlzylinder gasdurchlässig verbunden ist, so dass der Raum zwischen der Zweikomponentenkartusche und den axial beweglichen Stößeln mit der umgebenden Atmosphäre gasdurchlässig verbunden ist.

Hierdurch wird verhindert, dass sich beim Vortreiben der Stößel und des ersten Kolbens im ersten Hohlzylinder ein Überdruck ausbilden kann, der dem Vortrieb der Stößel beziehungsweise des ersten Kolbens entgegenwirkt.

Gemäß einer bevorzugten Ausführung des Pasten-Applikationssystems kann auch vorgesehen sein, dass mindestens eine verschließbare Öffnung zur Befüllung mit Flüssigkeit vorgesehen ist, die mit dem Hohlraum für die Flüssigkeit verbunden ist, der von dem Flüssigkeitsbehälter, dem ersten Leitungsmittel, dem zweiten Leitungsmittel, dem ersten Rückschlagventil, dem zweiten Rückschlagventil, dem durch den ersten Kolben begrenzten Innenraum des ersten Hohlzylinders und dem durch den zweiten Kolben begrenzten Innenraum des zweiten Hohlzylinders gebildet ist, wobei bevorzugt die Öffnung zum Befüllen mit Flüssigkeit an dem Flüssigkeitsbehälter angeordnet ist.

Damit kann eine einfache Befüllung des gesamten hydraulischen Systems mit der Flüssigkeit gewährleistet werden.

Ferner kann vorgesehen sein, dass mindestens eine verschließbare Entlüftungsöffnung vorgesehen ist, die mit dem Hohlraum für die Flüssigkeit verbunden ist, der vom Flüssigkeitsbehälter, dem ersten Leitungsmittel, dem zweiten Leitungsmittel, dem ersten Rückschlagventil, dem zweiten Rückschlagventil, dem durch den ersten Kolben begrenzten Innenraum des ersten Hohlzylinders und dem durch den zweiten Kolben begrenzten Innenraum des zweiten Hohlzylinders gebildet ist.

Mit der Entlüftungsöffnung kann sichergestellt werden, dass möglichst wenig Luft in der Flüssigkeit verbleibt, die sich störend auf den Vortrieb der Stößel und damit der Paste auswirken würde.

Dabei kann vorgesehen sein, dass die geschlossene Stirnseite des ersten Hohlzylinders gewölbt ist und dass in der Wölbung die verschließbare Entlüftungsöffnung angeordnet ist.

In der Wölbung sammeln sich die Lufteinschlüsse, so dass die Lufteinschlüsse an dieser Stelle besonders einfach entfernt werden können.

Auch kann vorgesehen sein, dass die Zweikomponentenkartusche, der erste Hohlzylinder und der zweite Hohlzylinder in einem Gehäuse angeordnet sind, wobei bevorzugt das erste Leitungsmittel, das erste Rückschlagventil, das zweite Leitungsmittel und das zweite Rückschlagventil ebenfalls in dem Gehäuse angeordnet sind.

Hierdurch kann das gesamte Pasten-Applikationssystem als Einheit bedient und in einer Hand gehalten werden. Beweglich Teile sind dadurch nach außen abgeschlossen, so dass es nicht zu einer ungewollten Blockade sich bewegender Teile kommen kann.

Bevorzugte Pasten-Applikationssysteme können sich dadurch auszeichnen, dass die Flüssigkeit ohne visuell erkennbare Gasblasen in dem Hohlraum für die Flüssigkeit enthalten ist und/oder dass in der Flüssigkeit weniger als 5 Vol% Gas enthalten ist, bevorzugt weniger als 1 Vol% Gas enthalten ist.

Hierdurch kann sichergestellt werden, dass keine kompressiblen Gasblasen oder Lufteinschlüsse in der Flüssigkeit enthalten sind, die zum Einen ungewollt und ungenutzt Kraft aufnehmen und die sich zum anderen sich bei Wegfall der Krafteinwirkung entspannen und dadurch ein Nachlaufen der Paste bewirken, ohne dass manuell eine Kraft auf das Pasten-Applikationssystem ausgeübt wird und ohne dass ein weiterer Austrag gewünscht wäre.

Zur Aufnahme der entstehenden Kräfte kann vorgesehen sein, dass die Abstände zwischen dem Druckbehälter und der Außenwand der Zweikomponentenkartusche und zwischen dem Druckbehälter und der Außenwand des ersten Hohlzylinders kleiner als 100 µm sind, bevorzugt kleiner als 50 µm sind. Die Abstände müssen nicht vollflächig diese Abstände aufweisen, es kann je nach Beschaffenheit der Zweikomponentenkartusche und des ersten Hohlzylinders ausreichend sein, wenn die Abstände bereichsweise eingehalten werden.

Hierdurch wird sichergestellt, dass der erste Hohlzylinder und die Zweikomponentenkartusche sich nicht aufgrund des sehr hohen hydraulischen Drucks stark verformen können, da der Druckbehälter anliegt oder nahezu anliegend ist und dadurch alle Scher- und Torsionskräfte aufnehmen kann. Ohne diese Maßnahme können die Zweikomponentenkartusche und der erste Hohlzylinder nicht aus einfachem Kunststoff und/oder mit geringer Stärke aufgebaut werden.

Bevorzugte Pasten-Applikationssysteme können vorsehen, dass der Druckbehälter aus einem Material ausgewählt aus Aluminium, Aluminiumlegierungen und Hochleistungskunststoffen gefertigt ist. Ganz besonders bevorzugte Pasten-Applikationssysteme können vorsehen, dass der Druckbehälter aus einem Material ausgewählt aus Duroplasten, Polyamiden, Polyamide-co-imiden, Polysulfonen, Polyketonen und Polyetherketonen gefertigt ist. Letztere Kunststoffe werden als Hochleistungskunststoffe angesehen.

Hierdurch wird eine ausreichende Stabilität des Druckbehälters durch die Wahl des Materials erreicht.

Mit einer wiederverwendbaren Weiterbildung des erfindungsgemäßen Pasten-Applikationssystems kann vorgesehen sein, dass ein für Flüssigkeiten durchlässiges drittes Leitungsmittel und ein reversibel manuell verschließbares drittes Ventil ersten Hohlzylinder mit dem Flüssigkeitsbehälter verbinden. Bevorzugt ist der Hohlraum zwischen dem ersten Kolben und der Rückwand des ersten Hohlzylinders über das dritte Leitungsmittel und das dritte Ventil mit dem Flüssigkeitsbehälter verbunden.

Hierdurch kann erreicht werden, dass die Flüssigkeit nach der Anwendung des Pasten-Applikationssystems wieder aus dem ersten Hohlzylinder heraus in den Flüssigkeitsbehälter gedrückt werden kann, indem die Stößel mit dem ersten Kolben wieder in den ersten Hohlzylinder hineingedrückt werden. Durch diesen Aufbau wird das Pasten-Applikationssystem also wiederverwendbar. Die leere Zweikomponentenkartusche kann dazu einfach aus dem Pasten-Applikationssystem beziehungsweise dem Druckbehälter des Pasten-Applikationssystems herausgenommen werden und durch eine gefüllte Zweikomponentenkartusche ersetzt werden.

Es kann erfindungsgemäß auch vorgesehen sein, dass das Flächenverhältnis zwischen der Stirnfläche des ersten Kolbens zur Stirnfläche des zweiten Kolbens mindestens 10 zu 1 beträgt, bevorzugt mindestens 20 zu 1 beträgt, besonders bevorzugt mindestens 30 zu 1 beträgt.

Durch eine derartige Übersetzung wird eine hohe Kraftübertragung erreicht, mit der auch sehr zähe Pasten aus dem Pasten-Applikationssystem ausgetrieben werden können und/oder sehr starke Mischer mit den Komponenten beziehungsweise der Paste durchflossen werden können.

Des Weiteren kann vorgesehen sein, dass die Zweikomponentenkartusche zwei Kartuscheninnenräume umfasst, wobei die Förderkolben in den Kartuscheninnenräumen axial beweglich sind, wobei bevorzugt ein erster Kartuscheninnenraum einen zweiten Kartuscheninnenraum koaxial umgibt.

Hierdurch werden die beiden Komponenten in zwei separaten Kartuscheninnenräumen gelagert. Durch die koaxiale Anordnung wird ein gegen die auftretenden Drucke besonders stabile Konfiguration erreicht. Anstelle der koaxialen Anordnung können auch side-by-side-Kartuschen verwendet werden. Hierzu muss die Form des Druckbehälters an die Form der Zweikomponentenkartusche angepasst sein.

Es kann vorgesehen sein, dass die Kartuscheninnenräume an der den Förderkolben gegenüberliegenden Seite durch einen manuell lösbaren Verschluss getrennt sind oder trennbar sind, wobei bevorzugt der Verschluss in ein Innengewinde an der Vorderseite der Zweikomponentenkartusche eingeschraubt ist oder einschraubbar ist.

Hierdurch kann das Pasten-Applikationssystem auch gut zum Lagern der Ausgangskomponenten eines PMMA-Knochenzements eingesetzt werden, da deren Ausgangskomponenten streng voneinander getrennt aufbewahrt werden müssen, da ansonsten eine chemische Reaktion zu einer frühzeitigen Aushärtung des Zements führt, die das Pasten-Applikationssystem unbrauchbar machen würde.

Mit der Erfindung wird auch vorgeschlagen, dass der Druckbehälter an den Stirnseiten jeweils mindestens eine Öffnung aufweist, wobei bevorzugt an einer der Öffnungen zumindest ein Ventil zum Ablassen von Gas angeordnet ist.

Die erste Öffnung ist zum Austragen der Paste vorgesehen und die zumindest eine andere auf der gegenüberliegenden Stirnseite zum Ablassen von Luft aus der Flüssigkeit und/oder zum Einleiten der Flüssigkeit.

Die der Erfindung zugrundeliegenden Aufgaben werden auch gelöst durch ein Verfahren zum Mischen und Austreiben einer Paste mit den Schritten:
eine Flüssigkeit wird aus dem Flüssigkeitsbehälter durch das erste Rückschlagventil und das erste Leitungsmittel in den zweiten Hohlzylinder geleitet; anschließend wird durch einen manuell ausgeübten Druck auf den zweiten Kolben in dem zweiten Hohlzylinder die Flüssigkeit aus dem zweiten Hohlzylinder durch das zweite Rückschlagventil und das zweite Leitungsmittel in den ersten Hohlzylinder gedrückt, wobei durch die in den ersten Hohlzylinder hineingepresste Flüssigkeit der erste Kolben, mit einer größeren Stirnfläche zur Flüssigkeit als der zweite Kolben, in Richtung der Zweikomponentenkartusche gedrückt wird, wobei zumindest die zwei axial in der Zweikomponentenkartusche verschiebbaren Förderkolben durch den ersten Kolben oder durch die an dem ersten Kolben befestigten Stößel in zumindest zwei Kartuscheninnenräumen der Zweikomponentenkartusche vorgetrieben werden, wobei der Inhalt aus der Zweikomponentenkartusche ausgetrieben und anschließend vermischt wird und nach dem Vermischen appliziert wird.

Das Verfahren kann bevorzugt auch zum Lagern der Ausgangskomponenten geeignet sein. Dazu kann vorgesehen sein, dass beim Lagern der Ausgangskomponenten der Verschluss in die Zweikomponentenkartusche eingesetzt ist, wobei die zumindest zwei Ausgangskomponenten durch den Verschluss voneinander getrennt werden, und der Verschluss vor der Anwendung des Pasten-Applikationssystems entfernt wird. Bevorzugt wird anstatt des Verschlusses ein Austragsrohr in die Zweikomponentenkartusche eingesetzt, durch das die Paste appliziert wird.

Es kann vorgesehen sein, dass zur Umsetzung des Verfahrens ein erfindungsgemäßes Pasten-Applikationssystem verwendet wird.

Es kann auch vorgesehen sein, dass die beim Einpressen der Flüssigkeit in den ersten Hohlzylinder und die beim Vortreiben des ersten Kolbens und der Förderkolben in der Zweikomponenten-Kartusche auftretenden Kräfte durch einen Druckbehälter aufgenommen werden, der die Zweikomponentenkartusche und den ersten Hohlzylinder umschließt, bevorzugt an der Zweikomponentenkartusche und dem ersten Hohlzylinder anliegt. Der Druckbehälter ist bevorzugt im Wesentlichen zylindrisch. Der Druckbehälter weist bevorzugt einen bereichsweise zylindrischen Innenraum auf.

Ferner kann vorgesehen sein, dass der zweite Hohlzylinder wiederholt durch ein Rückstellen des zweiten Kolbens mit der Flüssigkeit aus dem Flüssigkeitsbehälter gefüllt wird, wobei bevorzugt das Rückstellen des zweiten Kolbens durch eine beim Eindrücken des zweiten Kolbens gespannte Druckfeder oder ein anderes Rückstellelement erfolgt.

Des Weiteren kann auch vorgesehen sein, dass beim Einleiten der Flüssigkeit aus dem Flüssigkeitsbehälter in den zweiten Hohlzylinder das erste Rückschlagventil geöffnet und das zweite Rückschlagventil geschlossen ist und beim Eindrücken der Flüssigkeit von dem zweiten Hohlzylinder in den ersten Hohlzylinder das erste Rückschlagventil geschlossen und das zweite Rückschlagventil geöffnet ist.

Der Erfindung liegt die überraschende Erkenntnis zugrunde, dass es durch den erfindungsgemäßen Aufbau gelingt ein für die hydraulische Anwendung ausreichend stabiles manuell getriebenes Pasten-Applikationssystem bereitzustellen, das eine kompakte und kostengünstige Bauweise aufweist und das zudem auch zum Lagern der Zementkomponenten geeignet ist.

Für die Implantation von künstlichen Knie- und Hüftgelenken sind üblicherweise Knochenzementmengen von 40 g bis 75 g notwendig. Eigene Versuche, bei denen zwei Zementpasten von je 60 g unter Verwendung eines statischen Mischen in einem Austragsrohr mit einem Innendurchmesser von 9 mm zu einem Zementteig vermischt wurden, zeigen, dass für die Bewegung der Zementpasten durch den statischen Mischer sehr hohe Drucke von 20 bar bis 45 bar notwendig sind. Eigene Versuche mit Kunststoffkartuschen und darin axial beweglichen Kolben ergaben, dass die Kunststoffkartuschen bei Druckbeanspruchung bei Drücken größer als 20 bar deutliche Verformungen ausbilden. Durch diese Verformungen kommt es zu Undichtigkeiten zwischen der Innenfläche der Kartuschen und den Förderkolben trotz Verwendung von elastischen Dichtringen. Dadurch ist ein Austritt von pastösem Material möglich. Deswegen wird im Rahmen der vorliegenden Erfindung ein vorzugsweise zylinderförmiger Druckbehälter vorgeschlagen, der die Zweikomponentenkartusche und den ersten Hohlzylinder umschließt und in der Lage ist die mechanischen Belastungen aufzunehmen.

In weiteren Versuchen wurde gefunden, dass es sehr schwierig ist, Zementkartuschen während des Austrags der Zementpasten, während des Vorschubs der Förderkolben durch Stößel, so zu lagern, dass sich die Zementkartuschen bei Innendrücken größer 20 bar nicht von den Stößeln und dem Stößelantrieb durch Verbiegung oder Verwindung entfernen. Eigene Versuche zeigten weiterhin, dass aus Aluminiumlegierungen gefertigte Zementkartuschen für Polymethylmethacrylat-Zementpasten ungeeignet sind, weil die Legierungen und die darin enthaltenden Legierungsbestandteile wie Kupfer und Mangan eine unerwünschte radikalische Polymerisationen währen der Lagerung der Zementpasten in der Zementkartusche bewirken. Nur in Zementkartuschen aus geeigneten Kunststoffen, wie Poly-acrylnitril-co-methylmethacrylat und Polybutylenterephthalat, sind Polymethylmethacrylat enthaltende Zementpasten ausreichend dauerhaft lagerstabil.

Im Folgenden wird ein allgemeines Ausführungsbeispiel der Erfindung beschrieben.

Ein beispielhaftes Pasten-Applikationssystem ist zusammengesetzt aus einer Zweikomponentenkartusche mit zwei axial verschiebbaren Kolben, einem Austragsrohr mit integriertem statischen Mischer, einem ersten Hohlzylinder mit einer offenen Stirnseite und einer geschlossenen Stirnseite, in dem sich ein axial beweglicher erster Kolben mit zwei daran befestigten Stößeln befindet, einem zweiten Hohlzylinder, der einem manuell verschiebbaren zweiten Kolben enthält, einen Flüssigkeitsbehälter mit einem axial verschiebbaren Förderkolben, wobei der Flüssigkeitsbehälter mit einem ersten Leitungsmittel und mit einem ersten Rückschlagventil mit dem zweiten Hohlzylinder flüssigkeitsdurchlässig verbunden ist, und der zweite Hohlzylinder mit einem zweiten Leitungsmittel und einem zweiten Rückschlagventil mit dem ersten Hohlzylinder flüssigkeitsdurchlässig verbunden ist.

Dabei sind die Zweikomponentenkartusche und der axial dahinter angeordnete Hohlzylinder mit axial beweglichen Stößeln in einem zylinderförmigen Druckbehälter mit einem Zug-Modul nach ISO 527 größer 1500 MPa angeordnet, wobei der Druckbehälter an den Stirnseiten jeweils mindestens eine Öffnung hat und eine Öffnung ist an der Mantelfläche so angeordnet, dass der Hohlraum zwischen der Zweikomponentenkartusche und der den axial beweglichen Stößeln mit der umgebenden Atmosphäre gasdurchlässig verbunden ist.

Ferner ist mindestens eine verschließbare Öffnung zur Befüllung mit Flüssigkeit vorgesehen, die mit dem Hohlraum verbunden ist, der vom Flüssigkeitsbehälter, dem ersten Leitungsmittel, dem ersten Rückschlagventil, dem ersten Hohlzylinder mit dem ersten Kolben, dem zweiten Verbindungsmittel, dem zweiten Rückschlagventil, dem zweiten Hohlzylinder und dem zweiten Kolben gebildet wird.

Zudem kann vorgesehen sein, dass mindestens eine verschließbare Entlüftungsöffnung angeordnet ist, die mit dem Hohlraum verbunden ist, der vom Flüssigkeitsbehälter, dem ersten Leitungsmittel, dem ersten Rückschlagventil, dem Hohlzylinder mit dem ersten Kolben, dem zweiten Verbindungsmittel, dem zweiten Rückschlagventil, dem zweiten Hohlzylinder und dem zweiten Kolben gebildet wird.

Des Weiteren kann vorgesehen sein, dass eine Flüssigkeit ohne visuell erkennbare Gasblasen in dem Hohlraum angeordnet ist, der vom Flüssigkeitsbehälter, dem ersten Leitungsmittel, dem ersten Rückschlagventil, dem ersten Hohlzylinder mit dem ersten Kolben, dem zweiten Verbindungsmittel, dem zweiten Rückschlagventil, dem zweiten Hohlzylinder und dem zweiten Kolben gebildet wird.

Zudem kann vorgesehen sein, dass die Zweikomponentenkartusche, der erste Hohlzylinder mit dem ersten Kolben und Stößeln, das erste Leitungsmittel, das erste Rückschlagventil, der zweite Hohlzylinder, der zweite Kolben, das zweite Leitungsmittel, das zweite Rückschlagventil in einem Gehäuse angeordnet sind.

Bei dem beispielhaften Pasten-Applikationssystem ist vorgesehen, dass die Abstände zwischen dem Druckbehälter und der Außenwand der Zweikomponentenkartusche und zwischen dem Druckbehälter und der Außenwand des ersten Hohlzylinders kleiner 100 µm sind.

Der Druckbehälter kann dabei bevorzugt aus Aluminium oder aus Aluminiumlegierungen oder aus Hochleistungskunststoffen gebildet sein, wobei Polyamide, Polyamide-co-imide, Polysulfone, Polyketone und Polyetherketone besonders bevorzugt sind. Die geschlossene Stirnseite des ersten Hohlzylinders ist gewölbt und in der Wölbung ist die verschließbare Entlüftungsöffnung angeordnet, damit Lufteinschlüsse in der Flüssigkeit nach dem Einfüllen leicht abgelassen werden können. Dazu ist die Öffnung zum Befüllen mit Flüssigkeit bevorzugt an dem Flüssigkeitsbehälter angeordnet.

Hinter dem Kolben des Flüssigkeitsbehälters ist eine gespannte Feder angeordnet, die den Kolben axial im Flüssigkeitsbehälter bewegt und Flüssigkeit durch das erste Leitungsmittel und durch das erste Rückschlagventil zum zweiten Hohlzylinder presst. Ein für Flüssigkeiten durchlässiges drittes Leitungsmittel und ein reversibel verschließbares drittes Ventil kann den Hohlraum im ersten Hohlzylinder mit dem Flüssigkeitsbehälter verbinden, um die Flüssigkeit wieder aus dem ersten Hohlzylinder mit dem ersten Kolben in den Flüssigkeitsbehälter pressen zu können und so das Pasten-Applikationssystem wiederverwendbar beziehungsweise wieder einsatzbereit zu machen. Es muss dann lediglich eine neue gefüllte Zweikomponentenkartusche eingesetzt werden.

Um den zum Auspressen notwendigen Druck zu erhalten ist vorgesehen, dass das Flächenverhältnis zwischen der Stirnfläche des ersten Kolbens zur Stirnfläche des zweiten Kolbens mindestens 20 zu 1 ist, und bevorzugt größer 30 zu 1 ist.

Im Folgenden werden weitere Ausführungsbeispiele der Erfindung anhand von sieben schematisch dargestellten Figuren erläutert, ohne jedoch dabei die Erfindung zu beschränken. Dabei zeigt:
- Figur 1:: eine schematische Querschnittansicht eines erfindungsgemäßen Pasten-Applikationssystems;
- Figur 2:: eine schematische Querschnittansicht eines zweiten erfindungsgemäßen Pasten-Applikationssystems;
- Figur 3:: eine schematische Querschnittansicht eines Teils des Pasten-Applikationssystems nach Figur 2 mit eingeschraubtem Verschluss;
- Figur 4:: eine schematische perspektivische Teilschnittansicht des Pasten-Applikationssystems nach Figur 2 und 3 mit geöffnetem Gehäuse;
- Figur 5:: eine schematische perspektivische Ansicht des Pasten-Applikationssystems nach den Figuren 2 bis 4 mit eingeschraubtem Verschluss;
- Figur 6:: eine schematische perspektivische Ansicht des Pasten-Applikationssystems nach den Figuren 2 bis 5 mit eingeschraubtem Austragsrohr; und
- Figur 7:: eine schematische Querschnittansicht eines dritten erfindungsgemäßen Pasten-Applikationssystems.

+In den Figuren werden teilweise für gleiche oder gleichartige Bauteile die gleichen Bezugszeichen verwendet, auch wenn es sich um unterschiedliche Pasten-Applikationssysteme handelt.

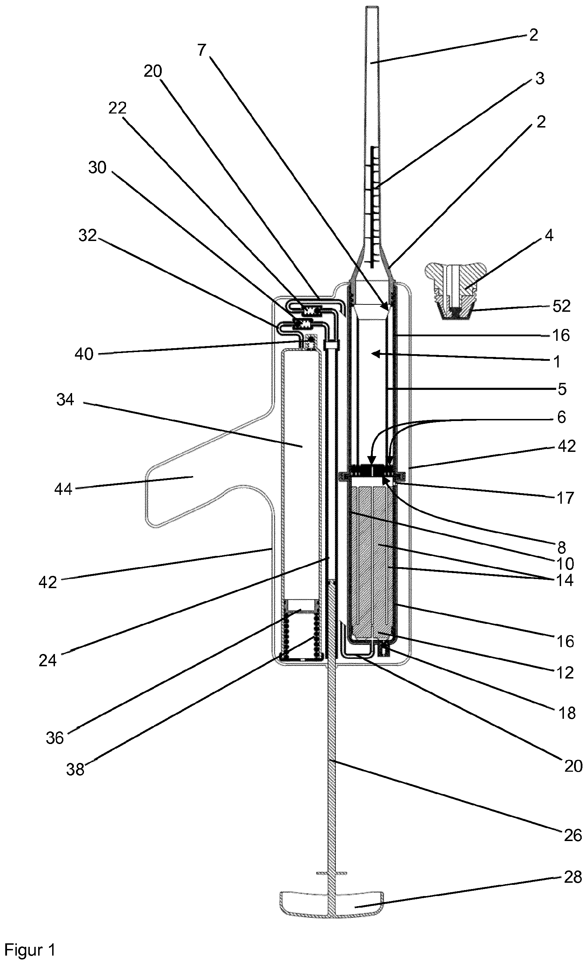

Figur 1 zeigt eine schematische Querschnittansicht eines erfindungsgemäßen Pasten-Applikationssystems. Das Pasten-Applikationssystem hat eine Zweikomponentenkartusche 1, in der in zwei voneinander getrennten und koaxial zueinander angeordneten Kartuscheninnenräumen zwei pastöse Ausgangskomponenten (nicht gezeigt) zur Herstellung eines Polymethylmethacrylat-Knochenzements (PMMA-Knochenzements) enthalten sind. An der Vorderseite der Zweikomponentenkartusche 1 ist ein Austragsrohr 2 befestigt, in dem ein statischer Mischer 3 angeordnet ist. Anstelle des Austragsrohrs 2 ist im Lagerungszustand des Pasten-Applikationssystems ein Verschluss 4 in das vordere Ende (in Figur 1 oben) der Zweikomponentenkartusche 1 eingesetzt.

Zur Trennung der Kartuscheninnenräume der Zweikomponentenkartusche 1 ist ein zylindrisches Rohr als Trennwand 5 zwischen den beiden Kartuscheninnenräumen vorgesehen. Dadurch ergibt sich ein äußerer Kartuscheninnenraum, der durch die zylindrische Trennwand 5 von einem inneren Kartuscheninnenraum getrennt ist. An dem rückseitigen Ende (in Figur 1 unten) der Zweikomponentenkartusche 1 sind zwei Förderkolben 6 in den Kartuscheninnenräumen angeordnet, die in den Kartuscheninnenräumen axial beweglich angeordnet sind und die über Dichtungsringe mit den Begrenzungswänden 5, 1 der Kartuscheninnenräume abgedichtet sind. Die Kartuscheninnenräume sind über Öffnungen 7 an der Vorderseite (in Figur 1 oben) miteinander verbunden, so dass die Kartuscheninnenräume zum Austragsrohr 2 hin ineinander münden. Die Zweikomponentenkartusche 1, das Austragrohr 2 und die Trennwand 5 können aus einem Kunststoff hergestellt werden, der chemisch gegenüber dem Kartuscheninhalt beständig ist.

Die Zweikomponentenkartusche 1 ist auf der Rückseite durch eine Folie 8 aus Kunststoff verschlossen, die die Zweikomponentenkartusche 1 gasdicht abschließt, wenn der Verschluss 4 in die Zweikomponentenkartusche 1 eingesetzt ist und die Öffnungen 7 verschließt.

An der Rückseite der Zweikomponentenkartusche 1 ist ein Hohlzylinder 10 angelegt, der auf der Rückseite (in Figur 1 unten) druckdicht durch einen Kolben 12 abgeschlossen ist. Der Kolben 12 ist mit Dichtungen gegen die Wandung des Hohlzylinders 10 abgedichtet und axial im Hohlzylinder 10 beweglich. Der Kolben 12 setzt sich in Stößel 14 fort, die zumindest in einem Bereich Ausnehmungen oder freie Bereiche aufweisen, wobei die Ausnehmungen notwendig sind, um die Stößel 14 in die Kartuscheninnenräume vortreiben zu können, das heißt, um die Trennwand 5 aufzunehmen.

Die Stößel 14 durchtrennen beim Vortreiben die Folie 8 und treiben die Förderkolben 6 in Richtung des Austragsrohrs 2, wodurch der Inhalt der Zweikomponentenkartusche 1 aus den Kartuscheninnenräumen ausgetrieben wird, in den Mischer 3 eingepresst wird, dort die beiden Ausgangskomponenten durchmischt werden und die gemischte Paste mit der Spitze des Austragsrohrs 2 appliziert werden kann.

Der Kolben 12 muss dazu aufgrund der hohen Zähigkeit der Ausgangskomponenten und der gemischten Paste und aufgrund des Widerstands durch den statischen Mischer 3 mit einem hohen Druck nach vorne (in Figur 1 nach oben) getrieben werden. Da auch der Hohlzylinder 10 aus einem Kunststoff besteht, müssen der Hohlzylinder 10 und die Zweikomponentenkartusche 1 vor den notwendigen, sehr großen Kräften geschützt werden. Hierzu sind die Zweikomponentenkartusche 1 und der Hohlzylinder 10 von einem Druckbehälter 16 aus Metall oder einem sehr zähen Kunststoff, wie beispielsweise Duroplaste, Polyamide, Polyamide-co-imide, Polysulfone, Polyketone und Polyetherketone, umgeben. Der Druckbehälter 16 ist in der Lage ein Zug-Modul nach EN ISO 527 größer 1500 MPa aufzunehmen. Der Druckbehälter 16 ist zweiteilig aufgebaut, wobei die beiden Teile des Druckbehälters 16 beispielsweise über ein Gewinde miteinander verbunden sind. Der Druckbehälter 16 ist innen zylindrisch und liegt außen an der Zweikomponentenkartusche 1 und dem Hohlzylinder 10 an oder hat nur einen sehr geringen Abstand von maximal 100 µm zu der Zweikomponentenkartusche 1 und dem Hohlzylinder 10. Hierdurch wird sichergestellt, dass sich die Zweikomponentenkartusche 1 und der Hohlzylinder 10 beim Vortreiben des Kolbens 12 nicht oder nur geringförmig verformen, so dass die beweglichen Teile, nämlich der Kolben 12, die Stößel 14 und die Förderkolben 6 nicht verkannten und blockieren und keine Paste außer durch das Austragsrohr 2 nach außen austreten kann.

An einer Seitenfläche sind in dem Hohlzylinder 10 und dem Druckbehälter 16 Öffnungen 17 vorgesehen, durch die Luft aus dem Zwischenraum im Hohlzylinder 10 zwischen dem Kolben 12 beziehungsweise den Stößeln 14 und der Folie 8 entweichen kann. Dadurch soll verhindert werden, dass das eingeschlossene Gas der Bewegung des Kolbens 12 entgegenwirkt. Auf der Rückseite des Hohlzylinders 10 und des Druckbehälters 16 ist ein manuell bedienbares Ventil 18 vorgesehen, mit dem Gas aus dem Raum zwischen der geschlossenen Rückwand des Hohlzylinders 10 und dem Kolben 12 abgelassen werden kann. Hierdurch kann ein Gas aus der hydraulischen Flüssigkeit abgelassen werden. Dazu ist die Öffnung für das Ventil 18 bevorzugt an einer Wölbung (nicht gezeigt) angeordnet, so dass, wenn das Pasten-Applikationssystem mit dem Austragsrohr 2 nach unten gehalten wird, sich die Luft oder andere Gaseinschlüsse in der Wölbung sammeln und mit dem Ventil 18 abgelassen werden können.

In die Rückseite des Hohlzylinders 10 mündet zudem eine Hydraulikleitung 20. In der Hydraulikleitung 20 ist ein Rückschlagventil 22 vorgesehen. Das Rückschlagventil 22 ist mit einer Kugel aufgebaut, die mit einer Druckfeder gelagert ist, wobei die Kugel mit der Druckfeder auf einen passenden Kugelsitz gepresst wird, um das Rückschlagventil 22 zu verschließen. Bei einer Strömung der enthaltenen Flüssigkeit mit ausreichendem Druck in Richtung des Hohlzylinders 10 öffnet das Rückschlagventil 22.

Die Hydraulikleitung 20 mündet in einen Hohlzylinder 24, in dem ein axial beweglich gelagerter Kolben 26 angeordnet ist. Der Kolben 26 ist über einen Dichtungsring gegen die Innenwände des Hohlzylinders 24 abgedichtet. Der Kolben 26 endet in einem Griff 28, mit dem der Kolben 26 manuell in den Hohlzylinder 24 einschiebbar und herausziehbar ist. Beim Einschieben des Kolbens 26 in den Hohlzylinder 24 wird die im Hohlzylinder 24 enthaltene Flüssigkeit durch die Hydraulikleitung 20 und das Rückschlagventil 22 in den Hohlzylinder 10 gepresst. Da die Stirnfläche des Kolbens 26 wesentlich kleiner ist als die korrespondierende Stirnfläche des Kolbens 12 (die Stirnfläche des Kolbens 26 ist mindestens um den Faktor zehn oder um den Faktor zwanzig kleiner als die Stirnfläche des Kolbens 12), wird hierdurch eine Übersetzung erreicht. Dadurch kann mit geringem Kraftaufwand durch einen Druck auf den Kolben 26 ein großer Druck auf den Kolben 12 ausgeübt werden, mit dem die Förderkolben 6 angetrieben werden, so dass sehr zähe Pasten ausgetrieben und gemischt werden können.

Der Hohlzylinder 24 mündet an der Vorderseite zusätzlich in eine Leitung 32, in der ein Rückschlagventil 30 angeordnet ist, das wie das andere Rückschlagventil 22 in der Hydraulikleitung 20 aufgebaut ist. Das Rückschlagventil 30 erlaubt nur eine Strömung der Flüssigkeit in Richtung des Hohlzylinders 24. Die Leitungen 20, 32, die Rückschlagventile 22, 30 und die Anschlüsse müssen die hohen auftretenden Drucke aufnehmen können, die beim Betrieb entstehen können, ohne dabei undicht zu werden.

Auf der anderen Seite mündet die Leitung 32 in einen zylindrischen Flüssigkeitsbehälter 34 mit einem im Vergleich zum zweiten Hohlzylinder 24 großen Volumen, der auf einer Seite mit einem axial beweglichen und abgedichteten Kolben 36 verschlossen ist. Der Kolben 36 ist mit einer Druckfeder 38 gespannt, so dass mit dem Kolben 36 die Flüssigkeit aus dem Flüssigkeitsbehälter 34 leicht in die Leitung 32 und in den Hohlzylinder 24 gedrückt beziehungsweise gezogen werden kann. Beim Zurückziehen des Kolbens 26 entsteht nämlich ein Unterdruck im Hohlzylinder 24, das Rückschlagventil 30 öffnet sich und die Flüssigkeit kann aus dem Flüssigkeitsbehälter 34 in den Hohlzylinder 24 strömen.

Der Kolben 12 wird also im Hohlzylinder 10 durch mehrmaliges Pumpen beziehungsweise durch mehrmaliges Drücken und Herausziehen des Kolbens 26 im Hohlzylinder 24 schubweise nach vorne getrieben.

Auf der Rückseite (in Figur 1 unten) ist in einer Kappe des Flüssigkeitsbehälters 34 eine Öffnung vorgesehen, damit sich zwischen dem Kolben 36 und der Kappe kein Unterdruck aufbauen kann, der einem Vortrieb des Kolbens 36 entgegenwirken würde. An der Vorderseite des Flüssigkeitsbehälters 34 ist ein Rückschlagventil 40 vorgesehen, durch das der Flüssigkeitsbehälter 34 mit Flüssigkeit gefüllt werden kann.

Die Hohlzylinder 10, 24, der Flüssigkeitsbehälter 34, die Zweikomponentenkartusche 1, die Leitungen 20, 32 und die Rückschlagventile 22, 30 sind in einem Gehäuse 42 aus Plastik angeordnet. Das Gehäuse 42 formt einen Pistolengriff 44 zum Halten des Pasten-Applikationssystems. Der Griff 28, das Ventil 18, und das Ventil 40 ragen aus dem Gehäuse 42 heraus beziehungsweise sind von außerhalb des Gehäuses 42 zugänglich.

Der Verschluss 4 weist Abdichtungen 52 in Form von passenden Flächen auf, die die Öffnungen 7 fluiddicht abschließen und so die Inhalte der Kartuscheninnenräume zuverlässig von einander trennen können. Der Verschluss 4 wird unmittelbar vor der Anwendung des Pasten-Applikationssystems aus der Vorderseite der Zweikomponentenkartusche 1 entfernt und anstelle des Verschlusses 4 das Austragsrohr 2 in der Halterung befestigt, die zuvor bereits zur Halterung des Verschlusses 4 genutzt wurde. Bevorzugt ist die Halterung an der Zweikomponentenkartusche 1 ein Gewinde, das in ein Gegengewinde am Verschluss 4 oder in ein Gegengewinde am Austragsrohr 2 greift. Mit dem Gewinde kann eine kraftschlüssige Anlage der Abdichtungen 52 auf den Öffnungen 7 der Zweikomponentenkartusche 1 erreicht werden.

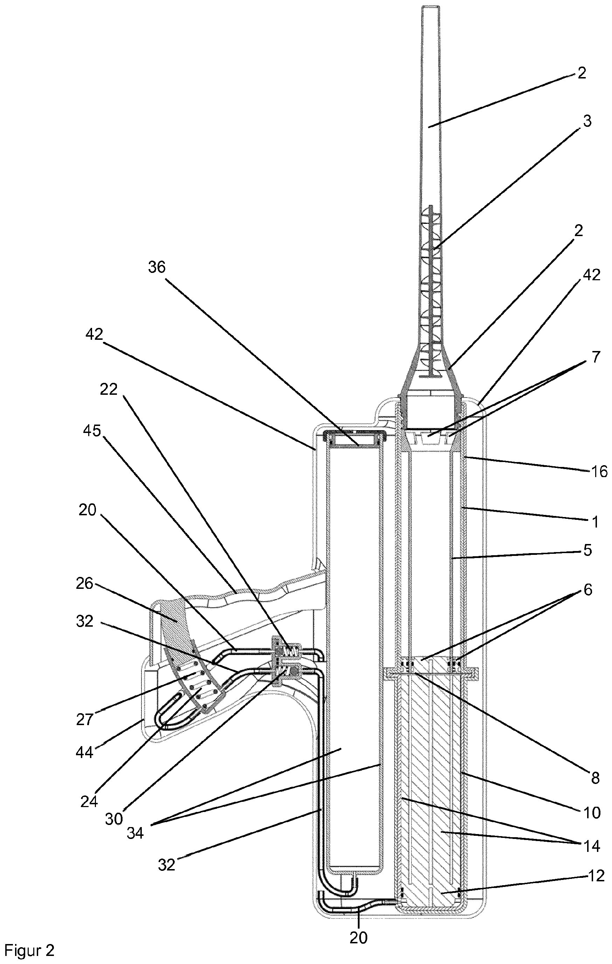

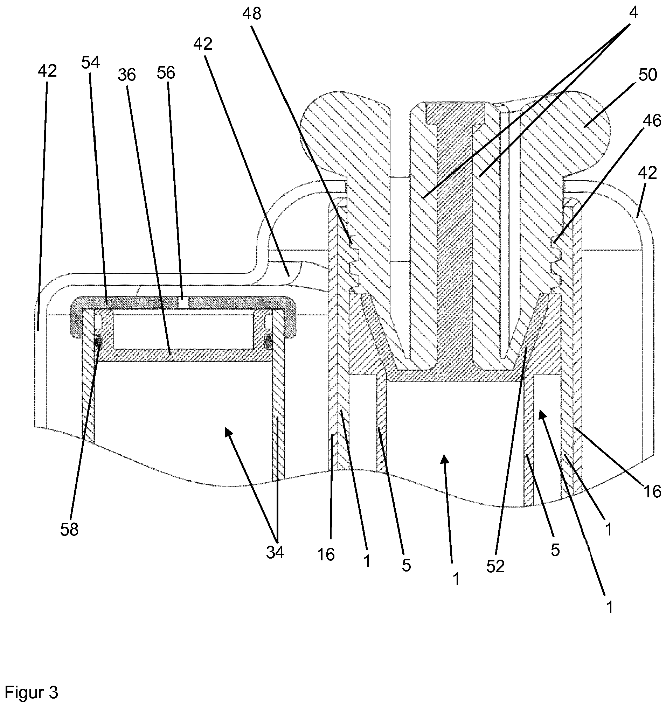

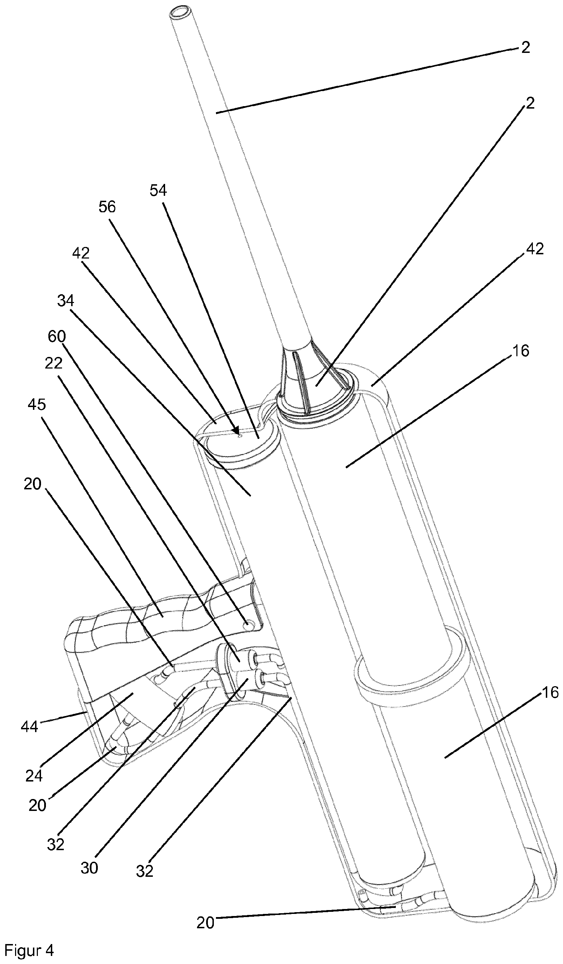

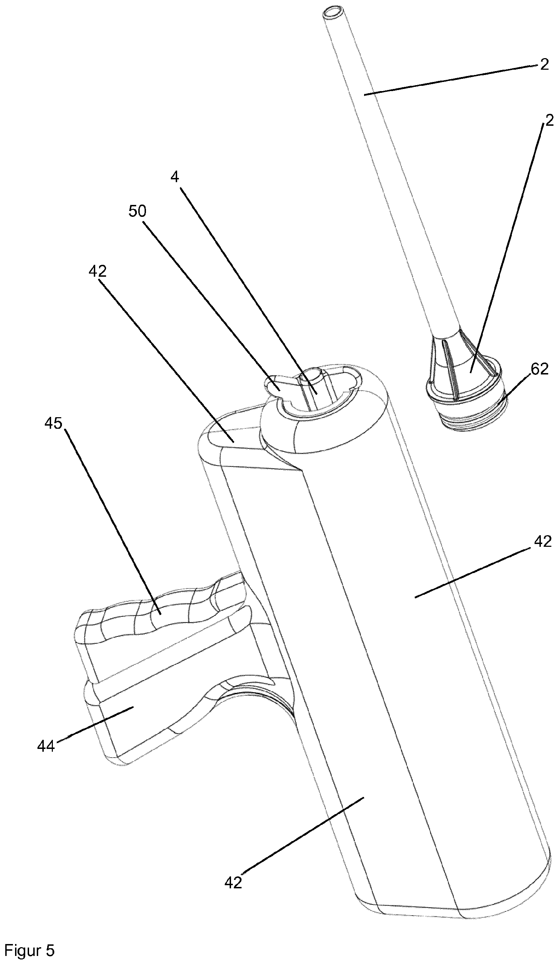

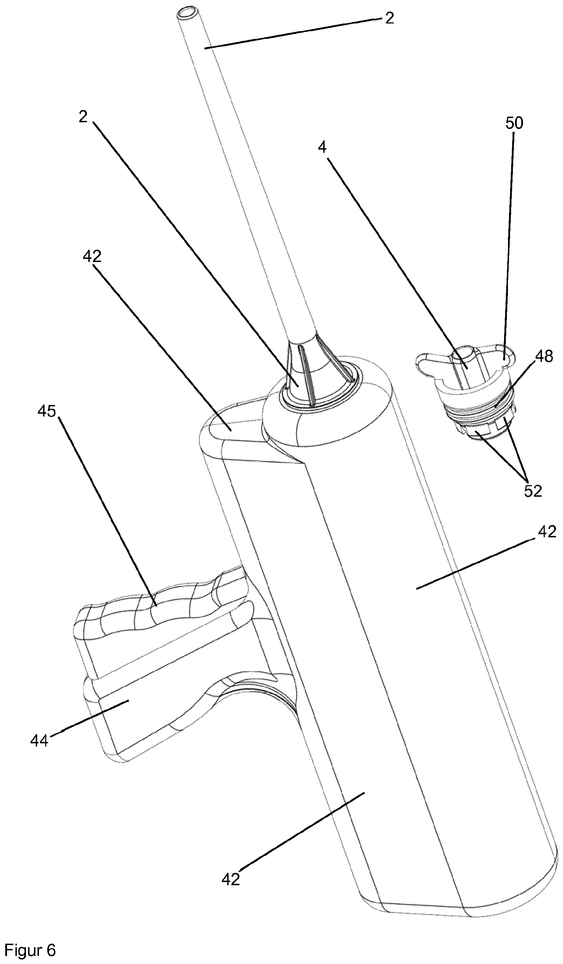

Die Figuren 2 bis 6 zeigen verschiedene schematische Ansichten eines zweiten erfindungsgemäßen Pasten-Applikationssystems mit einem geringfügig abweichenden Aufbau. Das Pasten-Applikationssystem hat eine Zweikomponentenkartusche 1, in der in zwei voneinander getrennten und koaxial zueinander angeordneten Kartuscheninnenräumen zwei pastöse Ausgangskomponenten (nicht gezeigt) zur Herstellung eines PMMA-Knochenzements enthalten sind. An der Vorderseite der Zweikomponentenkartusche 1 ist in den Figuren 2, 4 und 6 ein Austragsrohr 2 befestigt, in dem ein statischer Mischer 3 angeordnet ist. Anstelle des Austragsrohrs 2 ist im Lagerungszustand des Pasten-Applikationssystems, das in den Figuren 3 und 5 gezeigt ist, ein Verschluss 4 in das vordere Ende (in Figur 1 oben) der Zweikomponentenkartusche 1 eingesetzt.

Zur Trennung der Kartuscheninnenräume der Zweikomponentenkartusche 1 ist ein zylindrisches Rohr als Trennwand 5 zwischen den beiden Kartuscheninnenräumen vorgesehen. Dadurch ergibt sich ein äußerer Kartuscheninnenraum, der durch die zylindrische Trennwand 5 von einem inneren Kartuscheninnenraum getrennt ist. An dem rückseitigen Ende (in den Figuren 2 bis 6 unten) der Zweikomponentenkartusche 1 sind zwei Förderkolben 6 in den Kartuscheninnenräumen angeordnet, die in den Kartuscheninnenräumen axial beweglich angeordnet sind und die über Dichtungsringe mit den Begrenzungswänden 5, 1 der Kartuscheninnenräume abgedichtet sind. Die Kartuscheninnenräume sind über Öffnungen 7 an der Vorderseite (in Figur 2 oben) miteinander verbunden, so dass die Kartuscheninnenräume zum Austragsrohr 2 hin ineinander münden. Die Zweikomponentenkartusche 1, das Austragrohr 2 und die Trennwand 5 können aus einem Kunststoff hergestellt werden, der chemisch gegenüber dem Kartuscheninhalt beständig ist.

Die Zweikomponentenkartusche 1 ist auf der Rückseite durch eine Folie 8 aus metallbeschichtetem Kunststoff verschlossen, die die Zweikomponentenkartusche 1 gasdicht abschließt, wenn der Verschluss 4 in die Zweikomponentenkartusche 1 eingesetzt ist und die Öffnungen 7 verschließt.

An der Rückseite der Zweikomponentenkartusche 1 ist ein Hohlzylinder 10 angelegt, der auf der Rückseite (in den Figuren 2 bis 6 unten) druckdicht durch einen Kolben 12 abgeschlossen ist. Der Kolben 12 ist mit Dichtungen gegen die Wandung des Hohlzylinders 10 abgedichtet und axial im Hohlzylinder 10 beweglich. Der Kolben 12 setzt sich in Stößel 14 fort, die zumindest in einem Bereich Ausnehmungen oder freie Bereiche aufweisen, wobei die Ausnehmungen oder freien Bereiche notwendig sind, um die Stößel 14 in die Kartuscheninnenräume vortreiben zu können, das heißt, um die Trennwand 5 aufzunehmen.

Die Stößel 14 durchtrennen beim Vortreiben die Folie 8 und treiben die Förderkolben 6 in Richtung des Austragsrohrs 2, wodurch der Inhalt der Zweikomponentenkartusche 1 aus den Kartuscheninnenräumen ausgetrieben wird, in den Mischer 3 eingepresst wird, dort die beiden Ausgangskomponenten durchmischt werden und die gemischte Paste mit der Spitze des Austragsrohrs 2 appliziert werden kann.

Der Kolben 12 muss dazu aufgrund der hohen Zähigkeit der Ausgangskomponenten und der gemischten Paste und aufgrund des Widerstands durch den statischen Mischer 3 mit einem hohen Druck nach vorne (in den Figuren 2 bis 6 nach oben) getrieben werden. Da auch der Hohlzylinder 10 aus einem Kunststoff besteht, müssen der Hohlzylinder 10 und die Zweikomponentenkartusche 1 vor den notwendigen, sehr großen Kräften geschützt werden. Hierzu sind die Zweikomponentenkartusche 1 und der Hohlzylinder 10 von einem Druckbehälter 16 aus Metall oder einem sehr zähen Kunststoff, wie beispielsweise Duroplaste, Polyamide, Polyamide-co-imide, Polysulfone, Polyketone und Polyetherketone, umgeben. Der Druckbehälter 16 ist in der Lage ein Zug-Modul nach EN ISO 527 größer 1500 MPa aufzunehmen. Der Druckbehälter 16 ist zweiteilig aufgebaut, wobei die beiden Teile des Druckbehälters 16 beispielsweise über ein Gewinde miteinander verbunden sind. Der Druckbehälter 16 ist innen zylindrisch und liegt außen an der Zweikomponentenkartusche 1 und dem Hohlzylinder 10 an oder hat nur einen sehr geringen Abstand von maximal 100 µm zu der Zweikomponentenkartusche 1 und dem Hohlzylinder 10. Hierdurch wird sichergestellt, dass sich die Zweikomponentenkartusche 1 und der Hohlzylinder 10 beim Vortreiben des Kolbens 12 nicht oder nur geringförmig verformen, so dass die beweglichen Teile, nämlich der Kolben 12, die Stößel 14 und die Förderkolben 6 nicht verkannten und blockieren und keine Paste außer durch das Austragsrohr 2 nach außen austreten kann.

An einer Seitenfläche kann in dem Hohlzylinder 10 und dem Druckbehälter 16 optional eine Öffnung (nicht gezeigt) vorgesehen sein, durch die Luft aus dem Zwischenraum im Hohlzylinder 10 zwischen dem Kolben 12 beziehungsweise den Stößeln 14 und der Folie 8 entweichen kann. Dadurch kann gegebenenfalls verhindert werden, dass das eingeschlossene Gas der Bewegung des Kolbens 12 entgegenwirkt.

Auf der Rückseite des Hohlzylinders 10 und des Druckbehälters 16 ist ein manuell bedienbares Ventil 18 vorgesehen, mit dem Gas aus dem Raum zwischen der geschlossenen Rückwand des Hohlzylinders 10 und dem Kolben 12 abgelassen werden kann. Hierdurch kann ein Gas aus der hydraulischen Flüssigkeit abgelassen werden. Dazu ist die Öffnung für das Ventil 18 bevorzugt an einer Wölbung (nicht gezeigt) angeordnet, so dass, wenn das Pasten-Applikationssystem mit dem Austragsrohr 2 nach unten gehalten wird, sich die Luft oder andere Gaseinschlüsse in der Wölbung sammeln und mit dem Ventil 18 abgelassen werden können.

In die Rückseite des Hohlzylinders 10 mündet zudem eine Hydraulikleitung 20. In der Hydraulikleitung 20 ist ein Rückschlagventil 22 vorgesehen. Das Rückschlagventil 22 ist mit einer Kugel aufgebaut, die mit einer Druckfeder gelagert ist, wobei die Kugel mit der Druckfeder auf einen passenden Kugelsitz gepresst wird, um das Rückschlagventil 22 zu verschließen. Bei einer Strömung der enthaltenen Flüssigkeit mit ausreichendem Druck in Richtung des Hohlzylinders 10 öffnet das Rückschlagventil 22.

Die Hydraulikleitung 20 mündet in einen gebogenen (gekrümmten) Hohlzylinder 24, in dem ein beweglich gelagerter und gleichartig gebogener Kolben 26 angeordnet ist. Der Kolben 26 ist über einen Dichtungsring gegen die Innenwände des gebogenen Hohlzylinders 24 abgedichtet. Der Kolben 26 endet in einem Abzug 45, mit dem der Kolben 26 manuell in den gebogenen Hohlzylinder 24 eindrückbar und herausziehbar ist. Dazu ist der Abzug 45 um eine Achse 60 drehbar beziehungsweise kippbar gegen ein Gehäuse 42 des Pasten-Applikationssystems gelagert. Beim Einschieben des Kolbens 26 in den gebogenen Hohlzylinder 24 wird die im Hohlzylinder 24 enthaltene Flüssigkeit durch die Hydraulikleitung 20 und das Rückschlagventil 22 in den Hohlzylinder 10 gepresst. Da die Stirnfläche des Kolbens 26 wesentlich kleiner ist als die korrespondierende Stirnfläche des Kolbens 12, wird hierdurch eine Übersetzung erreicht. Dadurch kann mit geringem Kraftaufwand durch einen Druck auf den Kolben 26 ein großer Druck auf den Kolben 12 ausgeübt werden, mit dem die Förderkolben 6 angetrieben werden, so dass sehr zähe Pasten ausgetrieben und gemischt werden können. In dem gebogenen Hohlzylinder 24 ist eine Druckfeder 27 angeordnet, die nach dem Eindrücken des Abzugs 45 und damit des Kolbens 26 eine Rückstellung des Abzugs 45 und des Kolbens 26 bewirkt.

Der gebogene Hohlzylinder 24 mündet an der Vorderseite zusätzlich in eine Leitung 32, in der ein Rückschlagventil 30 angeordnet ist, das wie das andere Rückschlagventil 22 in der anderen Hydraulikleitung 20 aufgebaut ist. Das Rückschlagventil 30 erlaubt nur eine Strömung der Flüssigkeit in Richtung des Hohlzylinders 24. Die Leitungen 20, 32, die Rückschlagventile 22, 30 und die Anschlüsse müssen die hohen auftretenden Drucke aufnehmen können, die beim Betrieb entstehen können, ohne dabei undicht zu werden.

Auf der anderen Seite mündet die Leitung 32 in einen zylindrischen Flüssigkeitsbehälter 34 mit einem im Vergleich zum zweiten Hohlzylinder 24 großen Volumen, der auf einer Seite mit einem axial beweglichen und abgedichteten Kolben 36 verschlossen ist. Beim Rückstellen des Kolbens 26 mit der Druckfeder 27 entsteht ein Unterdruck im Hohlzylinder 24, das Rückschlagventil 30 öffnet sich und die Flüssigkeit kann aus dem Flüssigkeitsbehälter 34 in den Hohlzylinder 24 strömen.

Der Kolben 12 wird im Hohlzylinder 10 also durch mehrmaliges Pumpen beziehungsweise durch mehrmaliges Drücken des Abzugs 45 und damit des Kolbens 26 im gebogenen Hohlzylinder 24 schubweise nach vorne getrieben.

Auf der Rückseite (in Figur 1 unten) ist in einer Kappe 54 des Flüssigkeitsbehälters 34 eine Öffnung 56 vorgesehen, damit sich zwischen dem Kolben 36 und der Kappe 54 kein Unterdruck aufbauen kann, der einem Vortrieb des Kolbens 36 entgegenwirken würde. An der Vorderseite des Flüssigkeitsbehälters 34 ist ein Einlassventil (nicht gezeigt) vorgesehen, durch das der Flüssigkeitsbehälter 34 mit Flüssigkeit gefüllt werden kann.

Die Hohlzylinder 10, 24, der Flüssigkeitsbehälter 34, die Zweikomponentenkartusche 1, die Leitungen 20, 32 und die Rückschlagventile 22, 30 sind in dem Gehäuse 42 aus Plastik angeordnet. Das Gehäuse 42 formt einen Pistolengriff 44 zum Halten des Pasten-Applikationssystems. Der Abzug 45 und das Einlassventil ragen aus dem Gehäuse 42 heraus beziehungsweise sind von außerhalb des Gehäuses 42 zugänglich.

In Figur 3 ist eine schematische Querschnittansicht eines Teils des zweiten erfindungsgemäßen Pasten-Applikationssystems dargestellt. Das Pasten-Applikationssystem beziehungsweise die Zweikomponentenkartusche 1 ist dabei auf der Vorderseite mit einem Verschluss 4 verschlossen, der die beiden Kartuscheninnenräume voneinander trennt. Die Zweikomponentenkartusche 1 ist dazu an der Vorderseite mit einem Innengewinde 46 ausgestattet, in das ein Außengewinde 48 des Verschlusses 4 eingeschraubt ist. Der Verschluss 4 ist mit Hilfe von Flügel 50 nach Art einer Flügelmutter schraubbar. Dichtungsflächen 52 aus einem elastischen Kunststoff dichten die Öffnungen 7 (in Figur 3 nicht zu sehen) ab und trennen so die Kartuscheninnenräume voneinander. Dadurch ist eine ungewollte vorzeitige Reaktion der beiden Ausgangskomponenten ausgeschlossen.

Der Flüssigkeitsbehälter 34 ist auf einer Seite mit einer Kappe 54 verschlossen, in der eine Öffnung 56 vorgesehen ist. Mit der Öffnung 56 soll erreicht werden, dass sich kein Unterdruck zwischen der Kappe 54 und dem Kolben 36 als Volumenausgleichselement ausbilden kann, der einer weiteren Bewegung des Kolbens 34 ins Innere des Flüssigkeitsbehälters 34 entgegenwirken könnte. Vorteilhafterweise kann zwischen der Kappe 54 und dem Kolben 36 eine gespannte Druckfeder (nicht gezeigt) angeordnet sein, um die Bewegung des Kolbens 36 ins Innere des Flüssigkeitsbehälters 34 zu unterstützen.

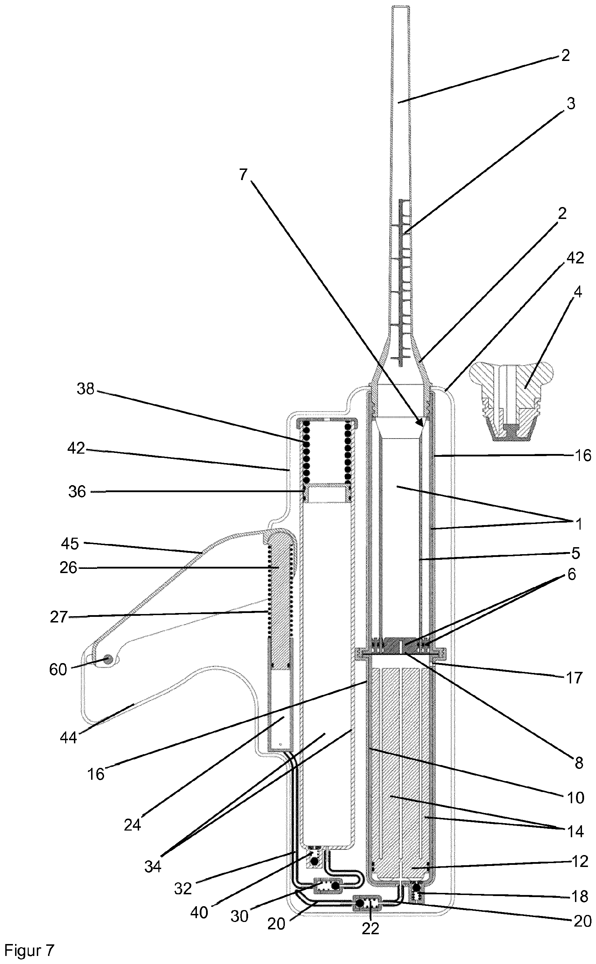

Figur 7 zeigt eine schematische Querschnittansicht eines dritten erfindungsgemäßen Pasten-Applikationssystems, das in einigen wenigen Einzelheiten von den ersten beiden Pasten-Applikationssystemen abweicht. Das dritte Pasten-Applikationssystem hat ebenfalls eine Zweikomponentenkartusche 1, in der in zwei voneinander getrennten und koaxial zueinander angeordneten Kartuscheninnenräumen zwei pastöse Ausgangskomponenten (nicht gezeigt) zur Herstellung eines PMMA-Knochenzements enthalten sind. An der Vorderseite der Zweikomponentenkartusche 1 ist ein Austragsrohr 2 befestigt, in dem ein statischer Mischer 3 angeordnet ist.

Anstelle des Austragsrohrs 2 ist im Lagerungszustand des Pasten-Applikationssystems ein Verschluss 4 in das vordere Ende (in Figur 7 oben) der Zweikomponentenkartusche 1 eingesetzt.

Zur Trennung der Kartuscheninnenräume der Zweikomponentenkartusche 1 ist ein zylindrisches Rohr als Trennwand 5 zwischen den beiden Kartuscheninnenräumen vorgesehen. Dadurch ergibt sich ein äußerer Kartuscheninnenraum, der durch die zylindrische Trennwand 5 von einem inneren Kartuscheninnenraum getrennt ist. An dem rückseitigen Ende (in Figur 7 unten) der Zweikomponentenkartusche 1 sind zwei Förderkolben 6 in den Kartuscheninnenräumen angeordnet, die in den Kartuscheninnenräumen axial beweglich angeordnet sind und die über Dichtungsringe mit den Begrenzungswänden 5, 1 der Kartuscheninnenräume abgedichtet sind. Die Kartuscheninnenräume sind über Öffnungen 7 an der Vorderseite (in Figur 7 oben) miteinander verbunden, so dass die Kartuscheninnenräume zum Austragsrohr 2 hin ineinander münden. Die Zweikomponentenkartusche 1, das Austragrohr 2 und die Trennwand 5 können aus einem Kunststoff hergestellt werden, der chemisch gegenüber dem Kartuscheninhalt beständig ist.

Die Zweikomponentenkartusche 1 ist auf der Rückseite durch eine Folie 8 aus Kunststoff verschlossen, die die Zweikomponentenkartusche 1 gasdicht abschließt, wenn der Verschluss 4 in die Zweikomponentenkartusche 1 eingesetzt ist und die Öffnungen 7 verschließt.

An der Rückseite der Zweikomponentenkartusche 1 ist ein Hohlzylinder 10 angelegt, der auf der Rückseite (in Figur 7 unten) druckdicht durch einen Kolben 12 abgeschlossen ist. Der Kolben 12 ist mit Dichtungen gegen die Wandung des Hohlzylinders 10 abgedichtet und axial im Hohlzylinder 10 beweglich. Der Kolben 12 setzt sich in Stößel 14 fort, die zumindest in einem Bereich Ausnehmungen oder freie Bereiche aufweisen, wobei die Ausnehmungen oder freien Bereiche notwendig sind, um die Stößel 14 in die Kartuscheninnenräume vortreiben zu können, das heißt, um die Trennwand 5 aufzunehmen.

Die Stößel 14 durchtrennen beim Vortreiben die Folie 8 und treiben die Förderkolben 6 in Richtung des Austragsrohrs 2, wodurch der Inhalt der Zweikomponentenkartusche 1 aus den Kartuscheninnenräumen ausgetrieben wird, in den Mischer 3 eingepresst wird, dort die beiden Ausgangskomponenten durchmischt werden und die gemischte Paste mit der Spitze des Austragsrohrs 2 appliziert werden kann.

Der Kolben 12 muss dazu aufgrund der hohen Zähigkeit der Ausgangskomponenten und der gemischten Paste und aufgrund des Widerstands durch den statischen Mischer 3 mit einem hohen Druck nach vorne (in Figur 1 nach oben) getrieben werden. Da auch der Hohlzylinder 10 aus einem Kunststoff besteht, müssen der Hohlzylinder 10 und die Zweikomponentenkartusche 1 vor den dabei auftretenden sehr großen Kräften geschützt werden. Hierzu sind die Zweikomponentenkartusche 1 und der Hohlzylinder 10 von einem Druckbehälter 16 aus Metall oder einem sehr zähen Kunststoff, wie beispielsweise Duroplaste, Polyamide, Polyamide-co-imide, Polysulfone, Polyketone und Polyetherketone, umgeben. Der Druckbehälter 16 ist in der Lage ein Zug-Modul nach EN ISO 527 größer 1500 MPa aufzunehmen. Der Druckbehälter 16 ist zweiteilig aufgebaut, wobei die beiden Teile des Druckbehälters 16 beispielsweise über ein Gewinde oder einen Flansch mit einer Flanschspange miteinander verbunden sind. Der Druckbehälter 16 ist innen zylindrisch und liegt außen an der Zweikomponentenkartusche 1 und dem Hohlzylinder 10 an oder hat nur einen sehr geringen Abstand von maximal 100 µm zu der Zweikomponentenkartusche 1 und dem Hohlzylinder 10. Hierdurch wird sichergestellt, dass sich die Zweikomponentenkartusche 1 und der Hohlzylinder 10 beim Vortreiben des Kolbens 12 nicht oder nur geringförmig verformen, so dass die beweglichen Teile, nämlich der Kolben 12, die Stößel 14 und die Förderkolben 6 nicht verkannten und blockieren und keine Paste außer durch das Austragsrohr 2 nach außen austreten kann.

An einer Seitenfläche sind in dem Hohlzylinder 10 und dem Druckbehälter 16 Öffnungen 17 vorgesehen, durch die Luft aus dem Zwischenraum im Hohlzylinder 10 zwischen dem Kolben 12 beziehungsweise den Stößeln 14 und der Folie 8 entweichen kann. Dadurch soll verhindert werden, dass das eingeschlossene Gas der Bewegung des Kolbens 12 entgegenwirkt. Auf der Rückseite des Hohlzylinders 10 und des Druckbehälters 16 ist ein manuell bedienbares Ventil 18 vorgesehen, mit dem Gas aus dem Raum zwischen der geschlossenen Rückwand des Hohlzylinders 10 und dem Kolben 12 abgelassen werden kann. Hierdurch kann ein Gas aus der hydraulischen Flüssigkeit abgelassen werden. Dazu ist die Öffnung für das Ventil 18 bevorzugt an einer Wölbung (nicht gezeigt) angeordnet, so dass, wenn das Pasten-Applikationssystem mit dem Austragsrohr 2 nach unten gehalten wird, sich die Luft oder andere Gaseinschlüsse in der Wölbung sammeln und mit dem Ventil 18 abgelassen werden können. Das Ventil 18 kann auch als Sicherheitsventil verwendet werden, das bei zu großem Druck öffnet.

In die Rückseite des Hohlzylinders 10 mündet zudem eine Hydraulikleitung 20. In der Hydraulikleitung 20 ist ein Rückschlagventil 22 vorgesehen. Das Rückschlagventil 22 ist mit einer Kugel aufgebaut, die mit einer Druckfeder gelagert ist, wobei die Kugel mit der Druckfeder auf einen passenden Kugelsitz gepresst wird, um das Rückschlagventil 22 zu verschließen. Bei einer Strömung der enthaltenen Flüssigkeit mit ausreichendem Druck in Richtung des Hohlzylinders 10 öffnet das Rückschlagventil 22.

Die Hydraulikleitung 20 mündet in einen Hohlzylinder 24, in dem ein axial beweglich gelagerter Kolben 26 angeordnet ist. Der Kolben 26 ist über einen Dichtungsring gegen die Innenwände des Hohlzylinders 24 abgedichtet. Der Kolben 26 endet an einem Abzug 45, mit dem der Kolben 26 manuell in den Hohlzylinder 24 eindrückbar und herausziehbar ist. Dazu ist der Abzug 45 um eine Achse 60 drehbar beziehungsweise kippbar gegen ein Gehäuse 42 des Pasten-Applikationssystems gelagert. Beim Einschieben des Kolbens 26 in den Hohlzylinder 24 wird die im Hohlzylinder 24 enthaltene Flüssigkeit durch die Hydraulikleitung 20 und das Rückschlagventil 22 in den Hohlzylinder 10 gepresst. Da die Stirnfläche des Kolbens 26 wesentlich kleiner ist als die korrespondierende Stirnfläche des Kolbens 12 (die Stirnfläche des Kolbens 26 ist mindestens um den Faktor zehn oder mindestens um den Faktor zwanzig kleiner als die Stirnfläche des Kolbens 12), wird hierdurch eine Übersetzung erreicht. Dadurch kann mit geringem Kraftaufwand durch einen Druck auf den Kolben 26 ein großer Druck auf den Kolben 12 ausgeübt werden, mit dem die Förderkolben 6 angetrieben werden, so dass sehr zähe Pasten ausgetrieben und gemischt werden können. Der Kolben 26 ist mit einer Druckfeder 27 gegen den Hohlzylinder 24 gelagert, wobei die Druckfeder 27 nach dem Eindrücken des Abzugs 45 und damit des Kolbens 26 eine Rückstellung des Abzugs 45 und des Kolbens 26 bewirkt.

Der Hohlzylinder 24 mündet an der Vorderseite zusätzlich in eine Leitung 32, in der ein Rückschlagventil 30 angeordnet ist, das wie das andere Rückschlagventil 22 in der anderen Hydraulikleitung 20 aufgebaut ist. Das Rückschlagventil 30 erlaubt nur eine Strömung der Flüssigkeit in Richtung des Hohlzylinders 24. Die Leitungen 20, 32, die Rückschlagventile 22, 30 und die Anschlüsse müssen die hohen auftretenden Drucke aufnehmen können, die beim Betrieb entstehen können, ohne dabei undicht zu werden.

Auf der anderen Seite mündet die Leitung 32 in einen zylindrischen Flüssigkeitsbehälter 34 mit einem im Vergleich zum zweiten Hohlzylinder 24 großen Volumen, der auf einer Seite mit einem axial beweglichen und abgedichteten Kolben 36 verschlossen ist. Der Kolben 36 ist mit einer Druckfeder 38 gespannt, so dass mit dem Kolben 36 die Flüssigkeit aus dem Flüssigkeitsbehälter 34 leicht in die Leitung 32 und in den Hohlzylinder 24 gedrückt beziehungsweise gezogen werden kann. Beim Rückstellen des Kolbens 26 mit der Druckfeder 27 entsteht ein Unterdruck im Hohlzylinder 24, das Rückschlagventil 30 öffnet sich und die Flüssigkeit kann aus dem Flüssigkeitsbehälter 34 in den Hohlzylinder 24 strömen.

Der Kolben 12 wird im Hohlzylinder 10 also durch mehrmaliges Pumpen beziehungsweise durch mehrmaliges Drücken und Herausziehen des Kolbens 26 im Hohlzylinder 24 schubweise nach vorne getrieben.

Auf der Rückseite (in Figur 7 unten) ist in einer Kappe des Flüssigkeitsbehälters 34 eine Öffnung vorgesehen, damit sich zwischen dem Kolben 36 und der Kappe kein Unterdruck aufbauen kann, der einem Vortrieb des Kolbens 36 entgegenwirken würde. An der Vorderseite des Flüssigkeitsbehälters 34 ist ein Rückschlagventil 40 vorgesehen, durch das der Flüssigkeitsbehälter 34 mit Flüssigkeit gefüllt werden kann.

Die Hohlzylinder 10, 24, der Flüssigkeitsbehälter 34, die Zweikomponentenkartusche 1, die Leitungen 20, 32 und die Rückschlagventile 22, 30 sind in dem Gehäuse 42 aus Plastik angeordnet. Das Gehäuse 42 formt einen Pistolengriff 44 zum Halten des Pasten-Applikationssystems. Der Griff 28, das Ventil 18, und das Ventil 40 ragen aus dem Gehäuse 42 heraus beziehungsweise sind von außerhalb des Gehäuses 42 zugänglich.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Zweikomponentenkartusche
- 2: Austragsrohr
- 3: Statischer Mischer
- 4: Verschluss
- 5: Trennwand
- 6: Förderkolben
- 7: Öffnung der Zweikomponentenkartusche
- 8: Folie
- 10: Hohlzylinder
- 12: Kolben
- 14: Stößel
- 16: Druckbehälter
- 17: Öffnung
- 18: Ventil
- 20: Hydraulikleitung
- 22: Rückschlagventil
- 24: Hohlzylinder
- 26: Kolben
- 27: Feder
- 28: Griff
- 30: Rückschlagventil
- 32: Leitung
- 34: Flüssigkeitsbehälter
- 36: Kolben
- 38: Feder
- 40: Rückschlagventil
- 42: Gehäuse
- 44: Pistolengriff
- 45: Abzug
- 46: Innengewinde
- 48: Außengewinde
- 50: Flügel
- 52: Abdichtung
- 54: Kappe
- 56: Öffnung
- 58: Dichtung
- 60: Achse

## Patentansprüche

1. Pasten-Applikationssystem zum Lagern zweier Ausgangskomponenten, zum Mischen der Ausgangskomponenten zu einer Paste und zum Applizieren der Paste aufweisend
eine Zweikomponentenkartusche (1) mit zwei axial verschiebbaren Förderkolben (6),
einen ersten Hohlzylinder (10) mit einer offenen Stirnseite und einer zumindest teilweise geschlossenen Stirnseite, in dem sich ein axial beweglicher erster Kolben (12) mit zwei daran befestigten Stößeln (14) befindet und der mit der offenen Stirnseite axial an der Zweikomponentenkartusche (1) anliegend angeordnet ist,
einen zweiten Hohlzylinder (24), der einen manuell verschiebbaren zweiten Kolben (26) enthält, und
einen Flüssigkeitsbehälter (34), wobei der zweite Hohlzylinder (24) über ein erstes Leitungsmittel (32) und über ein erstes Rückschlagventil (30) mit dem Flüssigkeitsbehälter (34) flüssigkeitsdurchlässig verbunden oder verbindbar ist und der erste Hohlzylinder (10) über ein zweites Leitungsmittel (20) und über ein zweites Rückschlagventil (22) mit dem zweiten Hohlzylinder (24) flüssigkeitsdurchlässig verbunden oder verbindbar ist,
wobei die Zweikomponentenkartusche (1) und der axial benachbart angeordnete erste Hohlzylinder (10) mit den axial beweglichen Stößeln (14) in einem Druckbehälter (16) angeordnet sind und
wobei in dem Pasten-Applikationssystem ein Hohlraum für die Flüssigkeit durch den Flüssigkeitsbehälter (34), das erste Leitungsmittel (32), das zweite Leitungsmittel (20), das erste Rückschlagventil (30), das zweite Rückschlagventil (22), den durch den ersten Kolben (12) begrenzten Innenraum des ersten Hohlzylinders (10) und den durch den zweiten Kolben (26) begrenzten Innenraum des zweiten Hohlzylinders (24) gebildet ist und eine Flüssigkeit in dem Hohlraum für die Flüssigkeit enthalten ist oder in den Hohlraum für die Flüssigkeit einfüllbar ist.

2. Pasten-Applikationssystem nach Anspruch 1, **dadurch gekennzeichnet, dass** das Pasten-Applikationssystem ein Austragsrohr (2) mit einem integrierten statischen Mischer (3) aufweist, das an dem den Förderkolben (6) gegenüberliegenden Ende der Zweikomponentenkartusche (1) angeordnet ist oder zu befestigen ist.

3. Pasten-Applikationssystem nach Anspruch 1 oder 2, **dadurch gekennzeichnet, dass**
der Druckbehälter (16) ein Zug-Modul nach EN ISO 527 größer 1500 MPa aufweist.

4. Pasten-Applikationssystem nach einem der vorangehenden Ansprüche, **dadurch gekennzeichnet, dass**
der Flüssigkeitsbehälter (34) ein Volumenausgleichselement (36) aufweist, bevorzugt einen axial verschiebbaren dritten Kolben (36) als Volumenausgleichselement (36) aufweist.

5. Pasten-Applikationssystem nach Anspruch 4, **dadurch gekennzeichnet, dass** an der von dem Bereich für die Flüssigkeit abgewandten Seite des Volumenausgleichselements (36) des Flüssigkeitsbehälters (34), insbesondere an dem dritten Kolben (36), eine gespannte Druckfeder (38) angeordnet ist, die das Volumenausgleichselement (36) in Richtung des Flüssigkeitsbehälters (34) drückt, insbesondere den dritten Kolben (36) axial im Flüssigkeitsbehälter (34) bewegt, und Flüssigkeit durch das erste Leitungsmittel (32) und durch das erste Rückschlagventil (30) zum zweiten Hohlzylinder (24) presst.

6. Pasten-Applikationssystem nach einem der vorangehenden Ansprüche, **dadurch gekennzeichnet, dass**
eine Öffnung (17) in der Mantelfläche des Druckbehälters (16) vorgesehen ist, die mit einer Öffnung (17) im ersten Hohlzylinder (10) gasdurchlässig verbunden ist, so dass der Raum zwischen der Zweikomponentenkartusche (1) und den axial beweglichen Stößeln (14) mit der umgebenden Atmosphäre gasdurchlässig verbunden ist.

7. Pasten-Applikationssystem nach einem der vorangehenden Ansprüche, **dadurch gekennzeichnet, dass**
mindestens eine verschließbare Öffnung (40) zur Befüllung mit Flüssigkeit vorgesehen ist, die mit dem Hohlraum für die Flüssigkeit verbunden ist, der von dem Flüssigkeitsbehälter (34), dem ersten Leitungsmittel (32), dem zweiten Leitungsmittel (20), dem ersten Rückschlagventil (30), dem zweiten Rückschlagventil (22), dem durch den ersten Kolben (12) begrenzten Innenraum des ersten Hohlzylinders (10) und dem durch den zweiten Kolben (26) begrenzten Innenraum des zweiten Hohlzylinders (24) gebildet ist, wobei bevorzugt die Öffnung (40) zum Befüllen mit Flüssigkeit an dem Flüssigkeitsbehälter (34) angeordnet ist.

8. Pasten-Applikationssystem nach einem der vorangehenden Ansprüche, **dadurch gekennzeichnet, dass**
mindestens eine verschließbare Entlüftungsöffnung (18) vorgesehen ist, die mit dem Hohlraum für die Flüssigkeit verbunden ist, der vom Flüssigkeitsbehälter (34), dem ersten Leitungsmittel (32), dem zweiten Leitungsmittel (20), dem ersten Rückschlagventil (30), dem zweiten Rückschlagventil (22), dem durch den ersten Kolben (12) begrenzten Innenraum des ersten Hohlzylinders (10) und dem durch den zweiten Kolben (26) begrenzten Innenraum des zweiten Hohlzylinders (24) gebildet ist.

9. Pasten-Applikationssystem nach Anspruch 8, **dadurch gekennzeichnet, dass** die geschlossene Stirnseite des ersten Hohlzylinders (10) gewölbt ist und dass in der Wölbung die verschließbare Entlüftungsöffnung (18) angeordnet ist.

10. Pasten-Applikationssystem nach einem der vorangehenden Ansprüche, **dadurch gekennzeichnet, dass**
die Zweikomponentenkartusche (1), der erste Hohlzylinder (10) und der zweite Hohlzylinder (24) in einem Gehäuse (42) angeordnet sind, wobei bevorzugt das erste Leitungsmittel (32), das erste Rückschlagventil (30), das zweite Leitungsmittel (20) und das zweite Rückschlagventil (22) ebenfalls in dem Gehäuse (42) angeordnet sind.

11. Pasten-Applikationssystem nach einem der vorangehenden Ansprüche, **dadurch gekennzeichnet, dass**
die Flüssigkeit ohne visuell erkennbare Gasblasen in dem Hohlraum für die Flüssigkeit enthalten ist und/oder dass in der Flüssigkeit weniger als 5 Vol% Gas enthalten ist, bevorzugt weniger als 1 Vol% Gas enthalten ist.

12. Pasten-Applikationssystem nach einem der vorangehenden Ansprüche, **dadurch gekennzeichnet, dass**
die Abstände zwischen dem Druckbehälter (16) und der Außenwand der Zweikomponentenkartusche (1) und zwischen dem Druckbehälter (16) und der Außenwand des ersten Hohlzylinders (10) kleiner als 100 µm sind, bevorzugt kleiner als 50 µm sind.

13. Pasten-Applikationssystem nach einem der vorangehenden Ansprüche, **dadurch gekennzeichnet, dass**
der Druckbehälter (16) aus einem Material ausgewählt aus Aluminium, Aluminiumlegierungen und Hochleistungskunststoffen gefertigt ist, bevorzugt ausgewählt aus Duroplasten, Polyamiden, Polyamide-co-imiden, Polysulfonen, Polyketonen und Polyetherketonen gefertigt ist.

14. Pasten-Applikationssystem nach einem der vorangehenden Ansprüche, **dadurch gekennzeichnet, dass**
ein für Flüssigkeiten durchlässiges drittes Leitungsmittel und ein reversibel manuell verschließbares drittes Ventil den ersten Hohlzylinder (10) mit dem Flüssigkeitsbehälter (34) verbinden.

15. Pasten-Applikationssystem nach einem der vorangehenden Ansprüche, **dadurch gekennzeichnet, dass**
das Flächenverhältnis zwischen der Stirnfläche des ersten Kolbens (12) zur Stirnfläche des zweiten Kolbens (26) mindestens 10 zu 1 beträgt, bevorzugt mindestens 20 zu 1 beträgt, besonders bevorzugt mindestens 30 zu 1 beträgt.

16. Pasten-Applikationssystem nach einem der vorangehenden Ansprüche, **dadurch gekennzeichnet, dass**
die Zweikomponentenkartusche (1) zwei Kartuscheninnenräume umfasst, wobei die Förderkolben (6) in den Kartuscheninnenräumen axial beweglich sind, wobei bevorzugt ein erster Kartuscheninnenraum einen zweiten Kartuscheninnenraum koaxial umgibt.

17. Pasten-Applikationssystem nach Anspruch 16, **dadurch gekennzeichnet, dass** die Kartuscheninnenräume an der den Förderkolben (6) gegenüberliegenden Seite durch einen manuell lösbaren Verschluss (4) getrennt sind oder trennbar sind, wobei bevorzugt der Verschluss (4) in ein Innengewinde (46) an der Vorderseite der Zweikomponentenkartusche (1) eingeschraubt ist oder einschraubbar ist.

18. Pasten-Applikationssystem nach einem der vorangehenden Ansprüche, **dadurch gekennzeichnet, dass**
der Druckbehälter (16) an den Stirnseiten jeweils mindestens eine Öffnung aufweist, wobei bevorzugt an einer der Öffnungen zumindest ein Ventil (18) zum Ablassen von Gas angeordnet ist.

19. Verfahren zum Mischen und Austreiben einer Paste **gekennzeichnet durch** die Schritte:
eine Flüssigkeit wird aus dem Flüssigkeitsbehälter (34) **durch** das erste Rückschlagventil (30) und das erste Leitungsmittel (32) in den zweiten Hohlzylinder (24) geleitet;
anschließend wird **durch** einen manuell ausgeübten Druck auf den zweiten Kolben (26) in dem zweiten Hohlzylinder (24) die Flüssigkeit aus dem zweiten Hohlzylinder (24) **durch** das zweite Rückschlagventil (22) und das zweite Leitungsmittel (20) in den ersten Hohlzylinder (10) gedrückt, wobei **durch** die in den ersten Hohlzylinder (10) hineingepresste Flüssigkeit der erste Kolben (12), mit einer größeren Stirnfläche zur Flüssigkeit als der zweite Kolben (26), in Richtung der Zweikomponentenkartusche (1) gedrückt wird, wobei zumindest die zwei axial in der Zweikomponentenkartusche (1) verschiebbaren Förderkolben (6) **durch** den ersten Kolben (12) oder **durch** die an dem ersten Kolben (12) befestigten Stößel (14) in zumindest zwei Kartuscheninnenräumen der Zweikomponentenkartusche (1) vorgetrieben werden, wobei der Inhalt aus der Zweikomponentenkartusche (1) ausgetrieben und anschließend vermischt wird und nach dem Vermischen appliziert wird.

20. Verfahren nach Anspruch 19, **dadurch gekennzeichnet, dass** zur Umsetzung des Verfahrens ein Pasten-Applikationssystem nach einem der Ansprüche 1 bis 18 verwendet wird.

21. Verfahren nach Anspruch 19 oder 20, **dadurch gekennzeichnet, dass** die beim Einpressen der Flüssigkeit in den ersten Hohlzylinder (10) und die beim Vortreiben des ersten Kolbens (12) und der Förderkolben (6) in der Zweikomponenten-Kartusche auftretenden Kräfte durch einen Druckbehälter (16) aufgenommen werden, insbesondere durch einen im wesentlichen zylindrischen Druckbehälter (16) aufgenommen werden, der die Zweikomponenten-kartusche (1) und den ersten Hohlzylinder (10) umschließt, bevorzugt an der Zweikomponentenkartusche (1) und dem ersten Hohlzylinder (10) anliegt.

22. Verfahren nach einem der Ansprüche 19 bis 21, **dadurch gekennzeichnet, dass**
der zweite Hohlzylinder (24) wiederholt durch ein Rückstellen des zweiten Kolbens (26) mit der Flüssigkeit aus dem Flüssigkeitsbehälter (34) gefüllt wird, wobei bevorzugt das Rückstellen des zweiten Kolbens (26) durch eine beim Eindrücken des zweiten Kolbens (26) gespannte Druckfeder (27) oder ein anderes Rückstellelement erfolgt.

23. Verfahren nach einem der Ansprüche 19 bis 22, **dadurch gekennzeichnet, dass**
beim Einleiten der Flüssigkeit aus dem Flüssigkeitsbehälter (34) in den zweiten Hohlzylinder (24) das erste Rückschlagventil (30) geöffnet und das zweite Rückschlagventil (22) geschlossen ist und beim Eindrücken der Flüssigkeit von dem zweiten Hohlzylinder (24) in den ersten Hohlzylinder (10) das erste Rückschlagventil (30) geschlossen und das zweite Rückschlagventil (22) geöffnet ist.

## Claims

1. A paste application system for storing two basic components, for mixing the basic components to obtain a paste, and for applying the paste, comprising:
a two-component cartridge (1) having two axially slidable delivery pistons (6),
a first hollow cylinder (10) having an open face side and an at least partially closed face side, with an axially movable first piston (12) having two plungers (14) attached thereto being situated inside said first hollow cylinder (10) which, with its open face side, is arranged such that it axially rests on the two-component cartridge (1),
a second hollow cylinder (24) containing a manually slidable second piston (26), and
a fluid container (34), wherein the second hollow cylinder (24) is connected or can be connected to the fluid container (34) via a first conduction means (32) and via a first check valve (30) in a manner permeable to fluid, and wherein the first hollow cylinder (10) is connected or can be connected to the second hollow cylinder (24) via a second conduction means (20) and via a second check valve (22) in a manner permeable to fluid,
wherein the two-component cartridge (1) and the first hollow cylinder (10) that is arranged adjacent thereto in axial direction and has the axially movable plungers (14) are arranged in a pressure container (16), and
wherein a hollow space for the fluid is formed by the fluid container (34), the first conduction means (32), the second conduction means (20), the first check valve (30), the second check valve (22), the interior region of the first hollow cylinder (10) that is limited by the first piston (12) and the hollow space of the second hollow cylinder (24) that is limited by the second piston (26), and a fluid is contained in the hollow space for the fluid or can be filled into the hollow space for the fluid.

2. The paste application system in accordance with Claim 1, **characterised in that** the paste application system includes a discharge tube (2) having an integrated static mixer (3), wherein said discharge tube (2) is arranged at or is to be mounted to the end of the two-component cartridge (1) that is situated opposite to the delivery pistons (6).

3. The paste application system in accordance with Claim 1 or 2, **characterised in that**
the pressure container (16) has a modulus of tension pursuant to EN ISO 527 that is in excess of 1500 MPa.

4. The paste application system in accordance with any one of the preceding claims, **characterised in that**
the fluid container (34) has a volume compensation element (36), preferably an axially slidable third piston (36) as a volume compensation element (36).

5. The paste application system in accordance with Claim 4, **characterised in that** a loaded compression spring (38) is arranged on the side of the volume compensation element (36) of the fluid container (34) that is facing away from the area for the fluid, more particularly at the third piston (36), wherein the loaded compression spring (38) pushes the volume compensation element (36) towards the fluid container (34), more particularly moves the third piston (36) in the fluid container (34) in axial direction, and presses fluid through the first conduction means (32) and through the first check valve (30) to the second hollow cylinder (24).

6. The paste application system in accordance with any one of the preceding claims, **characterised in that**
an opening (17) is provided in the lateral surface of the pressure container (16), said opening (17) being connected to an opening (17) in the first hollow cylinder (10) in a manner permeable to gas, with the result that the space between the two-component cartridge (1) and the axially movable plungers (14) is connected to the ambient atmosphere in a manner permeable to gas.

7. The paste application system in accordance with any one of the preceding claims, **characterised in that**
at least one closable opening (40) is provided for being filled with fluid, said opening (40) being connected to the hollow space for the fluid, said hollow space being formed by the fluid container (34), the first conduction means (32), the second conduction means (20), the first check valve (30), the second check valve (22), the interior space of the first hollow cylinder (10) that is limited by the first piston (12) and the interior space of the second hollow cylinder (24) that is limited by the second piston (26), wherein the opening (40) for being filled with fluid is preferably arranged at the fluid container (34).

8. The paste application system in accordance with any one of the preceding claims, **characterised in that**
there is provided at least one closable ventilation opening (18) which is connected to the hollow space for the fluid, said hollow space being formed by the fluid container (34), the first conduction means (32), the second conduction means (20), the first check valve (30), the second check valve (22), the interior space of the first hollow cylinder (10) that is limited by the first piston (12) and the interior space of the second hollow cylinder (24) that is limited by the second piston (26).

9. The paste application system in accordance with Claim 8, **characterised in that** the closed face side of the first hollow cylinder (10) is curved and that the closable ventilation opening (18) is arranged in the curvature.

10. The paste application system in accordance any one of the preceding claims, **characterised in that**
the two-component cartridge (1), the first hollow cylinder (10) and the second hollow cylinder (24) are arranged in a housing (42), wherein the first conduction means (32), the first check valve (30), the second conduction means (20) and the second check valve (22) are preferably arranged in the housing (42) as well.

11. The paste application system in accordance with any one of the preceding claims, **characterised in that**
the fluid is contained in the hollow space for the fluid without any visually recognisable gas bubbles and/or that less than 5 per cent by volume of gas, preferably less than 1 per cent by volume of gas is contained in the fluid.

12. The paste application system in accordance with any one of the preceding claims, **characterised in that**
the distances between the pressure container (16) and the outer wall of the two-component cartridge (1) as well as between the pressure container (16) and the outer wall of the first hollow cylinder (10) are less than 100 µm, preferably less than 50 µm.

13. The paste application system in accordance with any one of the preceding claims, **characterised in that**
the pressure container (16) is made of a material that is selected from aluminium, aluminium alloys and high-performance plastics, preferably selected from thermosetting plastics, polyamides, polyamide-co-imides, polysulphones, polyketones and polyetherketones.

14. The paste application system in accordance with any one of the preceding claims, **characterised in that**
a third conduction means that is permeable to fluids and a third valve that can be closed manually in a reversible manner connect the first hollow cylinder (10) to the fluid container (34).

15. The paste application system in accordance with any one of the preceding claims, **characterised in that**
the area ratio between the face side of the first piston (12) to the face side of the second piston (26) is at least 10 to 1, preferably at least 20 to 1, most preferably at least 30 to 1.

16. The paste application system in accordance with any one of the preceding claims, **characterised in that**
the two-component cartridge (1) comprises two interior cartridge spaces, wherein the delivery pistons (6) in the interior cartridge spaces are movable in axial direction wherein, preferably, a first interior cartridge space coaxially surrounds a second interior cartridge space.

17. The paste application system in accordance with Claim 16, **characterised in that** the interior cartridge spaces are separated or can be separated by a manually releasable closure (4) on the side opposite to the delivery pistons (6) wherein, preferably, the closure (4) is screwed or can be screwed into an internal thread (46) on the front side of the two-component cartridge (1).

18. The paste application system in accordance with any one of the preceding claims, **characterised in that**
the pressure container (16) has at least one opening on each of the face sides wherein, preferably, at least one valve (18) for discharging gas is arranged at one of the openings.

19. A method for mixing and expelling a paste, **characterised by** the following steps:
a fluid is conducted out of the fluid container (34) through the first check valve (30) and the first conduction means (32) and into the second hollow cylinder (24),
subsequently, a pressure is manually exerted on the second piston (26) in the second hollow cylinder (24) to press the fluid out of the second hollow cylinder (24) through the second check valve (22) and the second conduction means (20) and into the first hollow cylinder (10), wherein the fluid pressed into the first hollow cylinder (10) causes the first piston (12) that has a larger face side towards the fluid than the second piston (26) to be pressed in the direction towards the two-component cartridge (1), wherein at least the two delivery pistons (6) that are axially slidable in the two-component cartridge (1) are advanced into at least two interior cartridge spaces of the two-component cartridge (1) by the first piston (12) or by the plungers (14) attached to the first piston (12), wherein the content is expelled from the two-component cartridge (1) and is subsequently mixed and is applied after having been mixed.

20. The method in accordance with Claim 19, **characterised in that** a paste application system in accordance with any one of Claims 1 to 18 is used to implement the method.

21. The method in accordance with Claim 19 or 20, **characterised in that**
the forces developing in the two-component cartridge while the fluid is pressed into the first hollow cylinder (10) and while the first piston (12) and the delivery pistons (6) are advanced are received by a pressure container (16), more particularly by an essentially cylindrical pressure container (16), said pressure container (16) enclosing the two-component cartridge (1) and the first hollow cylinder (10), preferably resting against the two-component cartridge (1) and the first hollow cylinder (10).

22. The method in accordance with any one of Claims 19 to 21, **characterised in that**
the second hollow cylinder (24) is repeatedly filled with the fluid from the fluid container (34) by resetting the second piston (26) wherein, preferably, the resetting of the second piston (26) is achieved by means of a compression spring (27) that is loaded when the second piston (26) is pushed in or by means of another resetting element.

23. The method in accordance with any one of Claims 19 to 22, **characterised in that**
when the fluid from the fluid container (34) is introduced into the second hollow cylinder (24), the first check valve (30) is opened and the second check valve (22) is closed and, when the fluid from the second hollow cylinder (24) is pressed into the first hollow cylinder (10), the first check valve (30) is closed and the second check valve (22) is opened.

## Revendications

1. Système d'application de pâte pour stocker deux composants de départ, pour mélanger les composants de départ en une pâte et pour appliquer la pâte, présentant une cartouche bi-composants (1) comprenant deux pistons de transport (6) mobiles axialement,
un premier cylindre creux (10) avec une face avant ouverte et une face avant au moins partiellement fermée, dans lequel se trouve un premier piston (12) mobile axialement avec deux poussoirs (14) fixés dessus et qui est disposé avec la face avant ouverte reposant axialement sur la cartouche bi-composants (1),
un deuxième cylindre creux (24) qui contient un deuxième piston (26) mobile manuellement, et
un récipient de liquide (34), dans lequel le deuxième cylindre creux (24) est relié ou peut être relié au récipient de liquide (34), en étant perméable aux liquides, par un premier moyen de conduit (32) et par un premier clapet anti-retour (30), et le premier cylindre creux (10) est relié ou peut être relié au deuxième cylindre creux (24), en étant perméable aux liquides, par un deuxième moyen de conduit (20) et par un deuxième clapet anti-retour (22), dans lequel la cartouche bi-composants (1) et le premier cylindre creux (10) disposé axialement dans le voisinage avec les poussoirs (14) mobiles axialement sont disposés dans un récipient sous pression (16) et
dans lequel, dans le système d'application de pâte, il est formé un espace creux pour le liquide par le récipient de liquide (34), le premier moyen de conduit (32), le deuxième moyen de liquide (20), le premier clapet anti-retour (30), le deuxième clapet anti-retour (22), l'intérieur du premier cylindre creux (10) délimité par le premier piston (12) et l'intérieur du deuxième cylindre creux (24) délimité par le piston (26) et un liquide est contenu dans l'espace creux pour le liquide ou peut être rempli dans l'espace creux pour le liquide.

2. Système d'application de pâte selon la revendication 1, **caractérisé en ce que** le système d'application de pâte présente un tube de distribution (2) avec un mélangeur statique intégré (3) qui est disposé ou est à fixer sur l'extrémité de la cartouche bi-composants (1) opposée aux pistons de transport (6).

3. Système d'application de pâte selon la revendication 1 ou 2, **caractérisé en ce que** le récipient sous pression (16) présente un module de traction supérieur à 1500 Mpa selon la norme EN ISO 527.

4. Système d'application de pâte selon l'une des revendications précédentes, **caractérisé en ce que** le récipient de liquide (34) présente un élément de compensation de volume (36), de préférence un troisième piston (36) mobile axialement en tant qu'élément de compensation de volume (36).

5. Système d'application de pâte selon la revendication 4, **caractérisé en ce qu'**un ressort de pression (38) contraint est disposé sur le côté de l'élément de compensation de volume (36) du récipient de liquide (34) détourné de l'espace pour le liquide, en particulier sur le troisième piston (36), qui appuie l'élément de compensation de volume (36) en direction du récipient de liquide (34), déplace en particulier le troisième piston (36) axialement dans le récipient de liquide (34) et presse du liquide à travers le premier moyen de conduit (32) et le premier clapet anti-retour (30) en direction du deuxième cylindre creux (24).

6. Système d'application de pâte selon l'une des revendications précédentes, **caractérisé en ce qu'**une ouverture (17) est prévue dans la surface d'enveloppe du récipient sous pression (16), qui est reliée à une ouverture (17) dans le premier cylindre creux (10), en étant perméable aux gaz, de sorte que l'espace entre la cartouche bi-composants (1) et les poussoirs (14) mobiles axialement est relié à l'atmosphère environnante en étant perméable aux gaz.

7. Système d'application de pâte selon l'une des revendications précédentes, **caractérisé en ce qu'**au moins une ouverture (40) pouvant être fermée est prévue pour le remplissage par du liquide, laquelle est reliée avec l'espace creux pour le liquide, qui est formé par le récipient de liquide (34), le premier moyen de conduit (32), le deuxième moyen de liquide (20), le premier clapet anti-retour (30), le deuxième clapet anti-retour (22), l'intérieur du premier cylindre creux (10) délimité par le premier piston (12) et l'intérieur du deuxième cylindre creux (24) délimité par le piston (26), dans lequel de préférence, l'ouverture (40) pour le remplissage par du liquide est disposée sur le récipient de liquide (34).

8. Système d'application de pâte selon l'une des revendications précédentes, **caractérisé en ce qu'**au moins une ouverture d'aération (18) pouvant être fermée est prévue qui est reliée avec l'espace creux pour le liquide, qui est formé par le récipient de liquide (34), le premier moyen de conduit (32), le deuxième moyen de liquide (20), le premier clapet anti-retour (30), le deuxième clapet anti-retour (22), l'intérieur du premier cylindre creux (10) délimité par le premier piston (12) et l'intérieur du deuxième cylindre creux (24) délimité par le deuxième piston (26).

9. Système d'application de pâte selon la revendication 8, **caractérisé en ce que** la face avant fermée du premier cylindre creux (10) est bombée et que l'ouverture d'aération (18) pouvant être fermée est disposée dans le bombement.

10. Système d'application de pâte selon l'une des revendications précédentes, **caractérisé en ce que** la cartouche bi-composants (1), le premier cylindre creux (10) et le deuxième cylindre creux (24) sont disposés dans un boîtier (42), dans lequel de préférence, le premier moyen de conduit (32), le premier clapet anti-retour (30), le deuxième moyen de conduit (20) et le deuxième clapet anti-retour (22) sont également disposés dans le boîtier (42).

11. Système d'application de pâte selon l'une des revendications précédentes, **caractérisé en ce que** le liquide est contenu dans l'espace creux pour le liquide sans bulles de gaz détectables visuellement et/ou que le liquide contient moins de 5 % en volume de gaz, de préférence moins de 1 % en volume de gaz.

12. Système d'application de pâte selon l'une des revendications précédentes, **caractérisé en ce que** les écarts entre le récipient sous pression (16) et la paroi extérieure de la cartouche bi-composants (1) et entre le récipient sous pression (16) et la paroi extérieure du premier cylindre creux (10) sont inférieurs à 100 µm, de préférence inférieurs à 50 µm.

13. Système d'application de pâte selon l'une des revendications précédentes, **caractérisé en ce que** le récipient sous pression (16) est fabriqué dans un matériau sélectionné parmi l'aluminium, les alliages d'aluminium et les plastiques haute performance, de préférence sélectionnés parmi les duroplastes, les polyamides, les polyamide-co-imides, les polysulfones, les polycétones, et les polyéthercétones.

14. Système d'application de pâte selon l'une des revendications précédentes, **caractérisé en ce qu'**un troisième moyen de conduit laissant passer les liquides et un troisième clapet pouvant être fermé manuellement réversible relient le premier cylindre creux (10) au récipient sous pression (34).

15. Système d'application de pâte selon l'une des revendications précédentes, **caractérisé en ce que** le rapport surfacique entre la face avant du premier piston (12) et la face avant du deuxième piston (26) est au moins de 10 à 1, de préférence d'au moins 20 à 1, particulièrement de préférence d'au moins 30 à 1.

16. Système d'application de pâte selon l'une des revendications précédentes, **caractérisé en ce que** la cartouche bi-composants (1) comprend deux espaces intérieurs de cartouche, dans lequel les pistons de transport (6) sont mobiles axialement dans les espaces intérieurs de cartouche, dans lequel de préférence, un premier espace intérieur de cartouche entoure coaxialement un deuxième espace de cartouche.

17. Système d'application de pâte selon la revendication 16, **caractérisé en ce que** les espaces intérieurs de cartouche sont séparés ou peuvent être séparés par une fermeture (4) séparable manuellement, sur le côté opposé aux pistons de transport (6), dans lequel de préférence, la fermeture (4) est vissée ou peut être vissée dans un filetage femelle (46) sur la face avant de la cartouche bi-composants (1).

18. Système d'application de pâte selon l'une des revendications précédentes, **caractérisé en ce que** le récipient sous pression (16) présente respectivement au moins une ouverture sur les faces avant, dans lequel de préférence au moins un clapet (18) pour évacuer du gaz est disposé sur une des ouvertures.

19. Procédé destiné au mélange et à la distribution d'une pâte, **caractérisé par** les étapes suivantes :
un liquide est guidé hors du récipient de liquide (34) par le premier clapet anti-retour (30) et le premier moyen de conduit (32) jusque dans le deuxième cylindre creux (24) ;
ensuite, par une pression exercée manuellement sur le deuxième piston (26) dans le deuxième cylindre creux (24), le liquide est pressé hors du deuxième cylindre creux (24) par le deuxième clapet anti-retour (22) et le deuxième moyen de conduit (20) jusque dans le premier cylindre creux (10), dans lequel, par le biais du liquide entré par pression dans le premier cylindre creux (10), le premier piston (12) est appuyé, avec une plus grande face avant vers le liquide que le deuxième piston (26), en direction de la cartouche bi-composants (1), dans lequel au moins les deux pistons de transport (6) mobiles axialement dans la cartouche bi-composants (1), sont avancés dans au moins deux espaces intérieurs de la cartouche bi-composants (1) par le premier piston (12) ou par les poussoirs (14) fixés sur le premier piston (12), dans lequel le contenu sort de la cartouche bi-composants (1) et est ensuite mélangé et appliqué après le mélange.

20. Procédé selon la revendication 19, **caractérisé en ce que** pour mettre en oeuvre le procédé, un système d'application de pâte selon l'une des revendications 1 à 18 est employé.

21. Procédé selon la revendication 19 ou 20, **caractérisé en ce que** les forces se produisant lors de l'entrée du liquide par pression dans le premier cylindre creux (10) et lors de l'avancée du premier piston (12) et des pistons de transport (6) dans la cartouche bi-composants sont absorbées par un récipient sous pression (16), en particulier absorbées par un récipient sous pression (16) essentiellement cylindrique, qui entoure la cartouche bi-composants (1) et le premier cylindre creux (10), repose de préférence sur la cartouche bi-composants (1) et le premier cylindre creux (10).

22. Procédé selon l'une des revendications 19 à 21, **caractérisé en ce que** le deuxième cylindre creux (24) est de nouveau rempli par un repositionnement du deuxième piston (26), avec le liquide hors du récipient de liquide (34), dans lequel de préférence, le repositionnement du deuxième piston (26) s'effectue par un ressort de pression (27) contraint par poussée du deuxième piston (26) ou un autre élément de repositionnement.

23. Procédé selon l'une des revendications 19 à 22, **caractérisé en ce que** lors de l'introduction du liquide hors du récipient sous pression (34) dans le deuxième cylindre creux (24), le premier clapet anti-retour (30) est ouvert et le deuxième clapet anti-retour (22) est fermé, et lors de la poussée du liquide du deuxième cylindre creux (24) dans le premier cylindre creux (10), le premier clapet anti-retour (30) est fermé et le deuxième clapet anti-retour (22) est ouvert.
